# EUROPEAN PATENT APPLICATION

(11) **EP 4 424 185 A1**
(43) Date of publication of application: **04.09.2024**
(21) Application number: 21962462.4
(22) Date of filing: 29.10.2021
(51) Int. Cl.: A24F 40/53, A24F 40/57

(54) **SUCTION DEVICE, SUBSTRATE, AND CONTROL METHOD OF SUCTION DEVICE**

(71) Applicant: Japan Tobacco Inc., Tokyo 105-6927 (JP)
(72) Inventor: MINATO, Junji, Tokyo 130-8603 (JP)
(74) Representative: Hoffmann Eitle
(86) International application number: PCT/JP2021/040020
(87) International publication number: WO 2023/073920

(57) **Abstract**

Provided is a suction device that enables highly accurate determination of puffing operations by a user using a simple method. This suction device is equipped with: a heating unit that heats a substrate including an aerosol source to generate an aerosol; a power supply unit that supplies electric power to the heating unit; a temperature measuring unit that measures the temperature of the heating unit; a memory unit that stores information in which a time-series transition of a target temperature of the heating unit is defined; and a control unit that controls the operation of the power supply so that the temperature of the heating unit reaches a target temperature, wherein the control unit determines the puffing operations by a user when a control value associated with the operation of the power supply satisfies predetermined fluctuating condition.

## Description

### TECHNICAL FIELD

The present invention relates to an inhaler, a base material, and a method for controlling an inhaler.

### BACKGROUND ART

Inhalers for generating substances, which are to be inhaled by users, have been spread widely. Examples of inhalers are electronic cigarettes and nebulizers. An inhaler such as that explained above generates, for example, flavor-component-added aerosol by using an aerosol source used for generating aerosol and a base material including a flavor source and so on used for adding flavor components to generated aerosol. A user can taste flavor by inhaling flavor-component-added aerosol generated by an inhaler.

In more detail, in an inhaler, for generating aerosol, a heating element performs operation to heat a base material in such a manner that the operation is performed in accordance with a heating profile that defines heating operation. The quality of experience that is gained when an inhaler is used is greatly affected by the heating profile. For example, Patent Literature 1 discloses a heating profile in which temperature increases and reaches the highest temperature right after a start of heating operation and gradually decreases thereafter. Further, Patent Literature 2 discloses a method for controlling electric power supplied to a heating element for maintaining the temperature of the heating element at target temperature.

### CITATION LIST

### PATENT LITERATURE

PTL 1: PCT international publication No. WO 2020/084773
PTL 2: PCT international publication No. WO 2013/098397

### SUMMARY OF INVENTION

### TECHNICAL PROBLEM

It is desired to improve the quality of experience that a user is provided with when an inhaler is used by the user.

Accordingly, the present disclosure is provided in view of the above matters; and an object of the present disclosure is to provide a construction that is able to improve the quality of experience that is provided when an inhaler is used (hereinafter, "inhalation experience").

### SOLUTION TO PROBLEM

For achieving the above object, an inhaler is provided according to an aspect of the present disclosure. The inhaler comprises: a heater for heating a base material, which includes an aerosol source, for generating aerosol; an electric power source for supplying electric power to the heater; a temperature measuring unit for measuring temperature of the heater; a memory for storing information that defines chronological change in target temperature of the heater; and a controller for controlling operation for supplying the electric power, for making the temperature of the heater reach the target temperature; wherein the controller makes a judgment indicating that a puff action has been performed by a user, in the case that a control value related to the operation for supplying the electric power has satisfied a predetermined change condition.

The target temperatures may be set to correspond to time sections, respectively, wherein each time section corresponds to the length of time elapsed since a start of the operation for supplying the electric power; and the controlling of the operation for supplying the electric power may comprise adjusting of the temperature of the heater to make the temperature of the heater reach the target temperature in an end stage in the corresponding time section.

The controlling of the operation for supplying the electric power may comprise feedback controlling of supplied electric power for compensating for change in the temperature of the heater.

The controlling of the operation for supplying the electric power may comprise adjusting of a duty ratio of an electric power pulse, according to pulse width modulation (PWM) control; and the control value may be the duty ratio of the electric power pulse.

The change condition may be a condition that the quantity of increase in the duty ratio is greater than a predetermined threshold value.

The quantity of increase in the duty ratio may be the ratio of increase in a moving average value with respect to chronological data of the duty ratio.

The predetermined threshold value may be set in such a manner that it becomes smaller as the length of time elapsed since a start of the operation for supplying the electric power becomes longer.

The change condition may be set in such a manner that it is related to the length of time elapsed since a start of the operation for supplying the electric power.

The chronological change in the target temperature may comprise first-kind chronological change and second-kind chronological change; the change condition comprises a first change condition and a second change condition; and the first change condition is related to the first-kind chronological change, and the second change condition is related to the second-kind chronological change.

The first change condition may be set as that more strict than the second change condition, in the case that the target temperature in the first-kind chronological change is set as that higher than the target temperature in the second-kind chronological change.

The controller may further be constructed to make a judgment indicating that a puff action has been performed, in the case that the temperature of the heater satisfies a predetermined temperature change condition.

The temperature measuring unit may be constructed to measure the temperature of the heater by using a temperature sensor arranged in a position near the heater.

The controller may further be constructed to make a judgment indicating that a puff action has been performed, in either one of or both the case that the control value has satisfied the predetermined change condition and the case that the temperature of the heater has satisfied the predetermined temperature change condition.

The chronological change may be related to a first section that corresponds to a period from a point in time when the operation for supplying the electric power starts to a point in time when a first length of time has elapsed, and a second section that corresponds to a period until a point in time when a second length of time has elapsed after the end of the first section; and the controller may be constructed to perform judgment, that is based on the temperature of the heater, in the first section to indicate that a puff action has been performed, in the case that the temperature of the heater has satisfied the predetermined temperature change condition, and perform judgment, that is based on the control value, in the second section to indicate that a puff action has been performed, in the case that the control value has satisfied the predetermined change condition.

In the chronological change, the first section may comprise an initial temperature-increasing section for preheating the heater after a start of the operation for supplying the electric power, and a middle temperature-decreasing section, that follows the initial temperature-increasing section, for lowering the temperature of the heater by stopping the operation for supplying the electric power; and the second section may comprise a temperature re-increasing section for again heating the heater by resuming the operation for supplying the electric power.

Further, for achieving the above object, the base material used in the above-explained inhaler is provided according to a different aspect of the present disclosure.

Further, for achieving the above object, a method for controlling an inhaler is provided according to a different aspect of the present disclosure. The inhaler comprises a heater for heating a base material, which includes an aerosol source, for generating aerosol, and an electric power source for supplying electric power to the heater; and the method includes measuring temperature of the heater, adjusting, based on information that defines chronological change in target temperature of the heater, a control value related to operation for supplying the electric power, for making the temperature of the heater reach the target temperature, and making a judgment indicating that a puff action has been performed by a user, in the case that a control value has satisfied a predetermined change condition, wherein the above steps are those performed during the operation for supplying the electric power.

The target temperatures may be set to correspond to time sections, respectively, wherein each time section corresponds to the length of time elapsed since a start of the operation for supplying the electric power; and the control value may be adjusted to make the temperature of the heater reach the target temperature in an end stage in the corresponding time section.

The control value may be a duty ratio of an electric power pulse; the adjusting the control value may comprise adjusting of the duty ratio according to pulse width modulation (PWM) control; and the change condition that is to be satisfied by the control value may be a condition that the quantity of increase in the duty ratio is greater than a predetermined threshold value.

The method may further comprise incrementing the counted number of times of puff actions performed by a user, in relation to making a judgment indicating that a puff action has been performed by a user, and terminating the operation for supplying the electric power when the counted number of times of puff actions has reached a predetermined number of times.

### ADVANTAGEOUS EFFECTS OF INVENTION

As explained above, according to the present disclosure, a construction that makes it possible to further improve the quality of experience, that is gained when an inhaler is used, is provided.

### BRIEF DESCRIPTION OF DRAWINGS

Fig. 1 is a schematic figure which schematically shows an example of a construction of an inhaler.
Fig. 2 is a figure which schematically shows a physical construction of an inhaler according to a present embodiment.
Fig. 3 is a perspective view of a heater assembly shown in Fig. 2.
Fig. 4 is a perspective view of a chamber.
Fig. 5 is a cross-section view of the chamber relating to arrows 4-4 shown in Fig. 4.
Fig. 6 is a cross-section view of the chamber relating to arrows 5-5 shown in Fig. 5.
Fig. 7 is a longitudinal cross-section view of the chamber which includes a non-pressing part and is in the state that a stick-type base material is being held in a holding part.
Fig. 8 is a longitudinal cross-section view of the chamber which includes a pressing part and is in the state that a stick-type base material is being held in the holding part.
Fig. 9 is a cross-section view of the chamber relating to arrows 7-7 shown in Fig. 8.
Fig. 10 is a graph which shows an example of chronological change in actual temperature of a heater 40 which was operated based on an example heat profile.
Fig. 11 is a flow chart showing an example of the flow of processing performed by the inhaler.
Fig. 12 is a graph which shows an example of chronological change in actual temperature of the heater 40 which was operated based on an example heat profile.
Fig. 13 is a block diagram which functionally shows a construction of a controller 116 which performs a control method according to a present embodiment.
Fig. 14 is a graph which shows an example of chronological change in the duty ratio in controlling of operation for supplying electric power.
Fig. 15 is an enlarged graph of the graph shown in Fig. 14.
Fig. 16 is an enlarged graph which shows an example of chronological change in the moving average of the duty ratio in operation for supplying electric power.
Fig. 17 is a flow chart which shows an example of the flow of control processing performed in a present embodiment.
Fig. 18 is a graph which shows examples of chronological change in actual temperature of the heater 40 which was operated based on modified-example heat profiles.
Fig. 19 is a flow chart which shows an example of the flow of control processing performed by using a modified example.

### DESCRIPTION OF EMBODIMENTS

In the following description, preferred embodiments of the present invention will be explained in detail with reference to the attached figures. It should be reminded that, in the present specification and the figures, a reference symbol that is the same as that assigned to one component is assigned to the other component if the function/construction thereof is the same as that of the one component, and overlapping explanation of the components is omitted thereby.

### « 1. Construction Example of Inhaler »

An inhaler is an example of a device which generates substances which are to be inhaled by a user. In the following description, it is supposed that a substance generated by an inhaler is aerosol. In a different embodiment, a substance generated by an inhaler may be a gas. In the following description, an action that is performed by a user for inhaling aerosol generated by an inhaler may simply be referred to as "inhalation" or "puff." Also, in the following description, an inhalation action performed by a user may also be referred to as a "puff action."

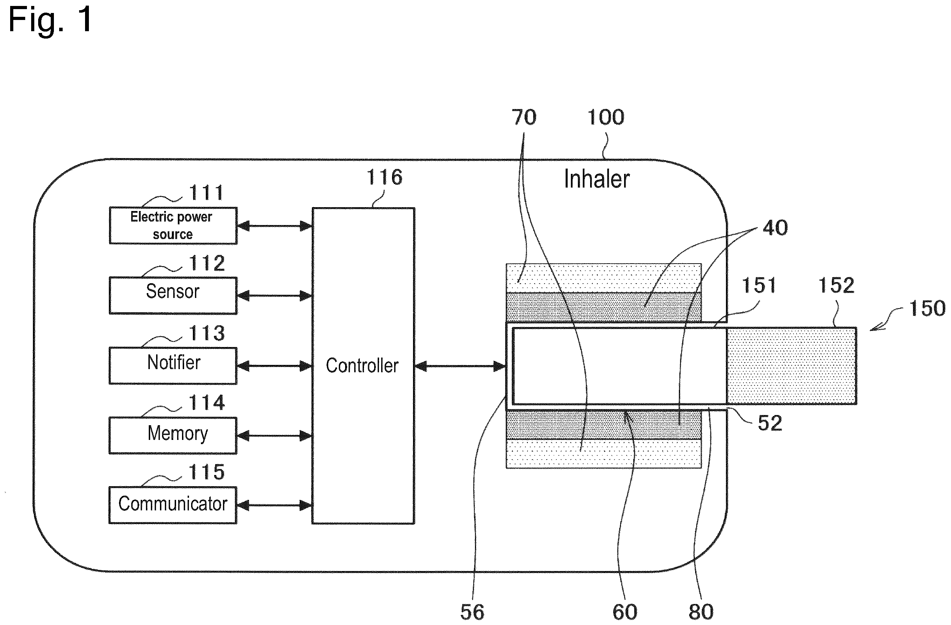

Fig. 1 is a schematic figure which schematically shows an example of a construction of an inhaler. As shown in Fig. 1, an inhaler 100 according to the present construction example comprises an electric power source 111, a sensor 112, a notifier 113, a memory 114, a communicator 115, a controller 116, a heater(s) 40, a holding part 60, and a heat insulator 70.

The electric power source 111 stores electric power. The electric power source 111 supplies, based on control by the controller 116, electric power to respective components in the inhaler 100. The electric power source 111 may comprise, for example, a rechargeable battery such as a lithium-ion secondary battery or the like.

The sensor 112 obtains various kinds of information relating to the inhaler 100. The sensor 112 may comprise a pressure sensor such as a microphone condenser or the like, a flow rate sensor, a temperature sensor, or the like, and obtains values relating to inhalation performed by a user. For example, by arranging a temperature sensor in a position adjacent to the heater 40, the sensor 112 constitutes a component of a temperature measuring unit which measures temperature of the heater 40. In a different example, the sensor 112 may comprise an input device such as a button, a switch, or the like which receives information input from a user.

The notifier 113 notifies a user of information. The notifier 113 may comprise, for example, a light emitting device for emitting light, a display device for displaying images, a sound outputting device for outputting sound, a vibration device for outputting vibration, or the like.

The memory 114 stores various kinds of information for operation of the inhaler 100. In the present embodiment, the memory 114 stores information that defines chronological change in target temperature that corresponds to the target values of the temperature of the heater 40. The memory 114 comprises, for example, a nonvolatile storage medium such as a flash memory or the like.

The communicator 115 is a communication interface which is able to perform communication operation conforming to an arbitrarily selected wired or wireless communication standard. For example, Wi-Fi (a registered trademark), Bluetooth (a registered trademark), or the like may be adopted as the communication standard.

The controller 116 functions as an arithmetic processing device and a control device, and controls overall operation of the inhaler 100 in accordance with various kinds of programs. For example, the controller 116 is realized by using an electronic circuit such as a CPU (Central Processing Unit), a microprocessor, or the like.

The holding part 60 holds a stick-type base material 150. The holding part 60 holds the stick-type base material 150 which is inserted into an inner space 80, that has been formed in the inhaler 100, from an opening 52 which makes the inner space 80 communicate with the outside. In this regard, the stick-type base material 150 held in the holding part 60 and the inhaler 100 comprising the holding part 60 constitute the inhalation system according to the present embodiment.

The stick-type base material 150, which is an example of the base material, comprises a base material part 151 and a suction opening part 152. The base material part 151 comprises an aerosol source. Aerosol is generated as a result that the aerosol source is atomized. The aerosol source is liquid such as polyhydric alcohol, such as glycerin, propylene glycol, or the like, or water, or the like, for example. The aerosol source may comprise a flavor component which is or is not originated from tobacco. In the case that the inhaler 100 is a medical inhaler such as a nebulizer or the like, the aerosol source may comprise a medicine. In this regard, the aerosol source is not limited to that in a liquid form, and it may be in a solid form. In the state that the stick-type base material 150 is being held by the holding part 60, at least a part of the base material part 151 is housed in the inside of the inner space 80, and at least a part of the suction opening part 152 protrudes from the opening 52. Thus, when the suction opening part 152, which protrudes from the opening 52, is held in a user's mouth and an inhalation action is performed by the user, the aerosol generated from the base material part 151 arrives at the inside of the user's mouth.

The heater 40 heats the aerosol source to atomize the aerosol source to thereby generate aerosol. For example, the heater 40 is constructed to have a film shape, and is arranged to cover the outer periphery of the holding part 60. Thus, when heat is generated by the heater 40, the base material part 151 of the stick-type base material 150 is heated from the outer periphery thereof, and aerosol is generated thereby. The heater 40 generates heat when electric power is supplied thereto from the electric power source 111.

The heat insulator 70 prevents heat transfer from the heater 40 to other components. For example, the heat insulator 70 comprises vacuum insulation material, aerogel insulation material, or the like.

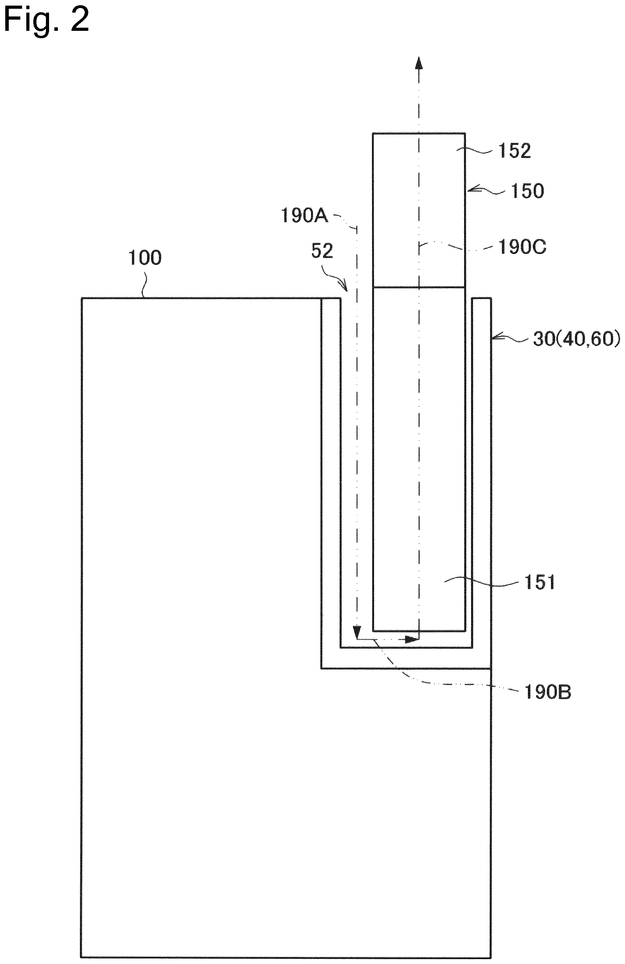

In the following description, details of the construction of the inhaler 100 will be explained with reference to Fig. 2 to Fig. 9. The inhaler 100 comprises a construction for heating the stick-type base material 150 while applying pressure thereto.

Fig. 2 is a figure which schematically shows a physical construction of the inhaler 100. As shown in Fig. 2, the inhaler 100 comprises a heater assembly 30 which comprises the heater(s) 40 and the holding part 60. As shown in Fig. 2, in the state that the stick-type base material 150 is being held in the heater assembly 30 (more specifically, the holding part 60), there is a space between the heater assembly 30 and the stick-type base material 150. When the stick-type base material 150 is held in a user's mouth and inhalation action is performed by the user, the air, that is taken form the opening 52, flows via the above space into the inside of the stick-type base material 150 from an end of the base material part 151, and further flows into the mouth of the user from an end of the suction opening part 152. That is, the flow of the air, that is to be inhaled by the user, comprises an air flow 190A, an air flow 190B, and an air flow 190C in this order; and the air, that is in the state that the aerosol generated in the stick-type base material 150 has been mixed therewith, is guided to the inside of the mouth of the user.

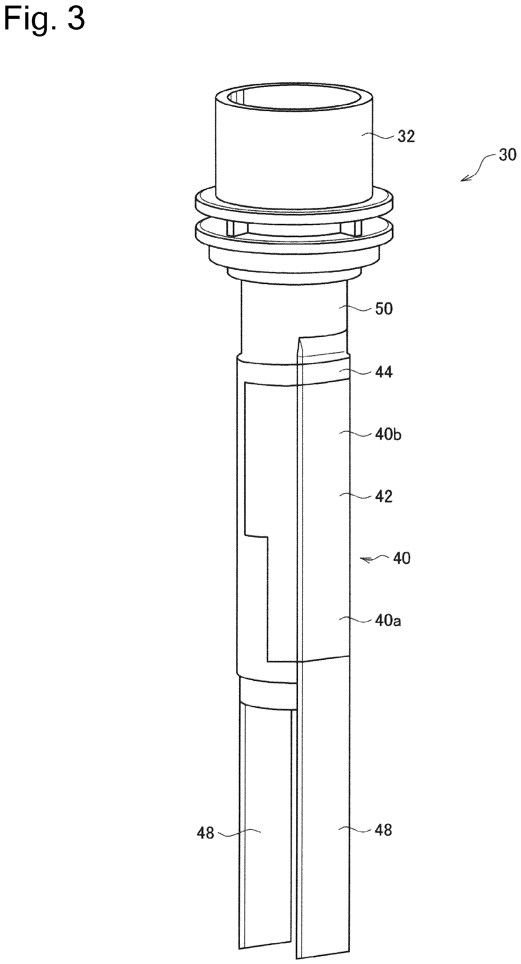

Fig. 3 is a perspective view of the heater assembly 30 shown in Fig. 2. As shown in Fig. 3, the heater assembly 30 comprises a top cap 32, the heater(s) 40, and a chamber 50. The chamber 50 is constructed to receive the stick-type base material 150. The heater 40 is constructed to heat the stick-type base material 150 received by the chamber 50. The top cap 32 may be constructed to have a function to guide the stick-type base material 150 when it is inserted into the chamber 50, and a function to fix the chamber 50 to the inhaler 100.

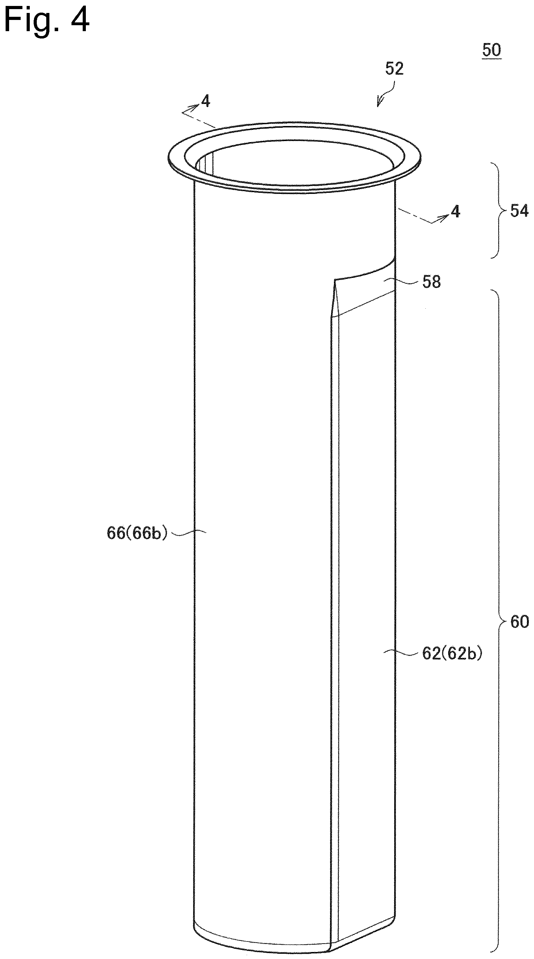

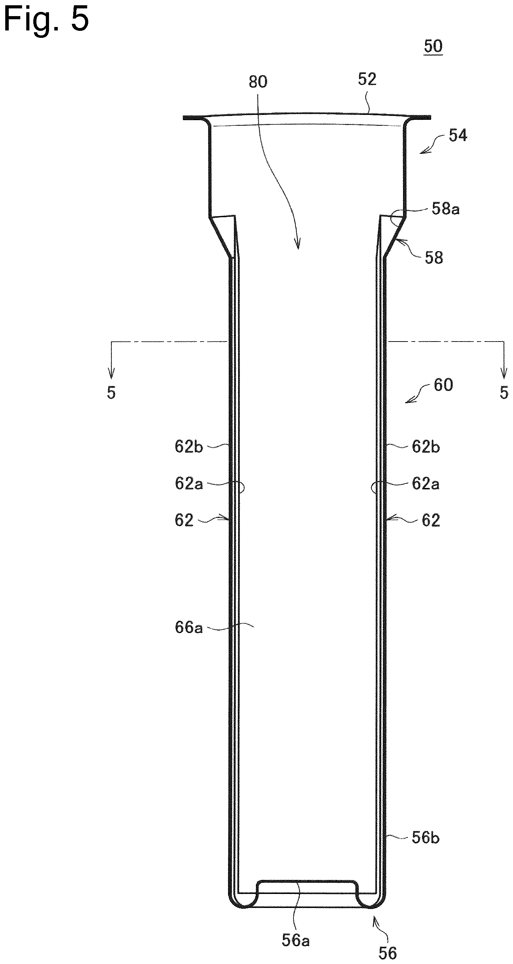

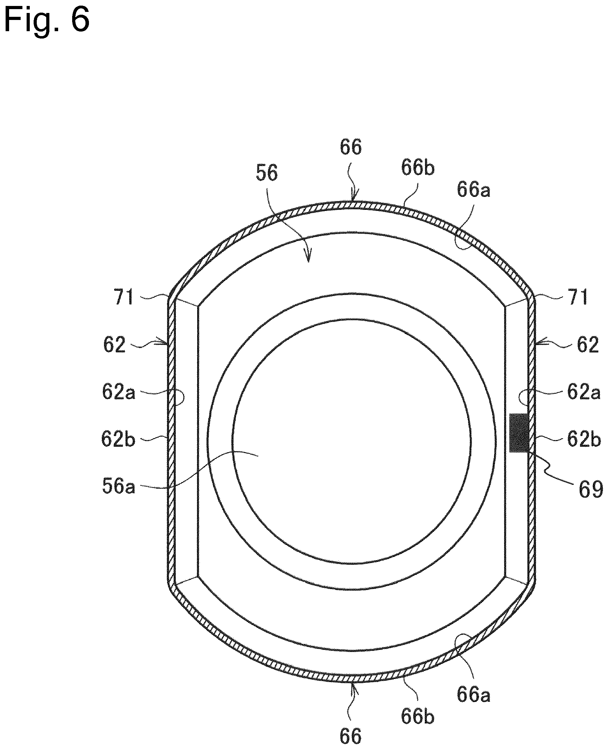

Fig. 4 is a perspective view of the chamber 50. Fig. 5 is a cross-section view of the chamber 50 and relates to arrows 4-4 shown in Fig. 4. Fig. 6 is a cross-section view of the chamber 50, and relates to arrows 5-5 shown in Fig. 5. As shown in Fig. 4 and Fig. 5, the chamber 50 comprises the opening 52, into which the stick-type base material 150 is to be inserted, and the holding part 60 for holding the stick-type base material 150. The chamber 50 is formed as a hollow member which surrounds the inner space 80 which receives the stick-type base material 150. The hollow member may be a bottomed cylindrical member. In this regard, the hollow member may be a non-bottomed cylindrical member. It may be preferable that the chamber 50 be constructed by using a metal having high thermal conductivity, and be formed by using stainless steel or the like. By adopting the above construction, it becomes possible to perform effective heating of the stick-type base material 150 from the chamber.

As shown in Fig. 5 and Fig. 6, the holding part 60 comprises pressing parts 62 for applying pressure to parts of the stick-type base material 150 and non-pressing parts 66. Each of the pressing parts 62 comprises an inner surface 62a and an outer surface 62b. Each of the non-pressing parts 66 comprises an inner surface 66a and an outer surface 66b. As shown in Fig. 3, the heater 40 is arranged on the outer surface 62b of the pressing part 62. It is preferable that the heater 40 be arranged in such a matter that no gap exists between it and the outer surface 62b of the pressing part 62.

It is preferable that the opening 52 of the chamber 50 be constructed to receive the stick-type base material 150 without applying pressure thereto. The opening 52 of the chamber 50, in a plane perpendicular to the longitudinal direction of the chamber, in other words, the direction of insertion of the stick-type base material 150 into the chamber 50 or the direction that the whole side surface of the chamber 50 extends, may have a polygon or elliptical shape; however, it is preferable that the opening 52 be constructed to have a circular shape.

As shown in each of Fig. 4, Fig. 5, and Fig. 6, the chamber 50 comprises two or more pressing parts 62 in positions in a circumferential direction in the chamber 50. As shown in each of Fig. 5, and Fig. 6, each one of the two pressing parts 62 in the holding part 60 faces the other of the two pressing parts 62. It is preferable that the length of at least a part of the space between the inner surfaces 62a of the two pressing parts 62 be shorter than the width of the part, that is to be positioned between the above part between the pressing parts 62, of the stick-type base material 150 which is to be inserted into the chamber 50. As shown in the figures, the inner surfaces 62a of the pressing parts 62 are plane surfaces.

As shown in Fig. 6, the inner surfaces 62a of the pressing parts 62 comprise a pair of planar pressing surfaces which are planar and face each other, and the inner surfaces 66a of the non-pressing parts 66 comprise a pair of curved non-pressing surfaces which are curved and face each other, wherein ends of the curved non-pressing surfaces are connected to ends of the planar pressing surfaces adjacent to the curved non-pressing surfaces, respectively. As shown in the figure, each of the curved non-pressing surfaces may have a cross section which generally has an arc shape, in a plane perpendicular to the longitudinal direction of the chamber 50. As shown in Fig. 6, the holding part 60 is constructed by using a metal cylindrical body having uniform thickness.

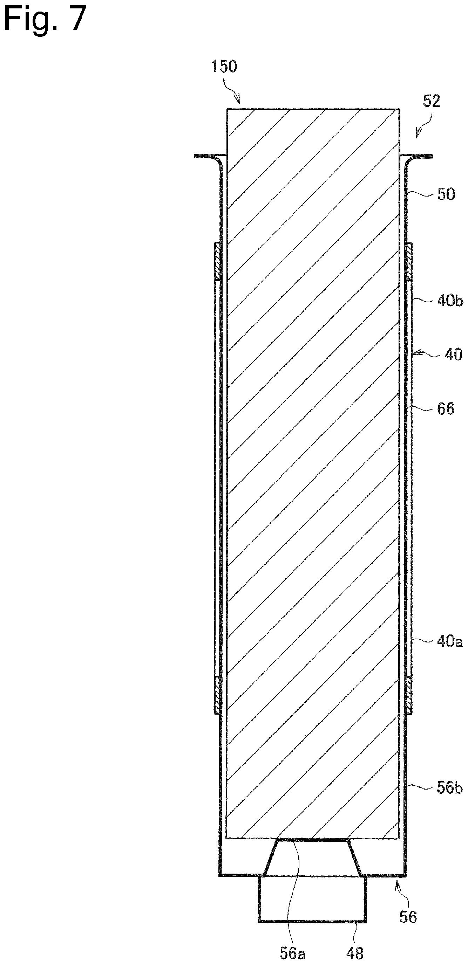

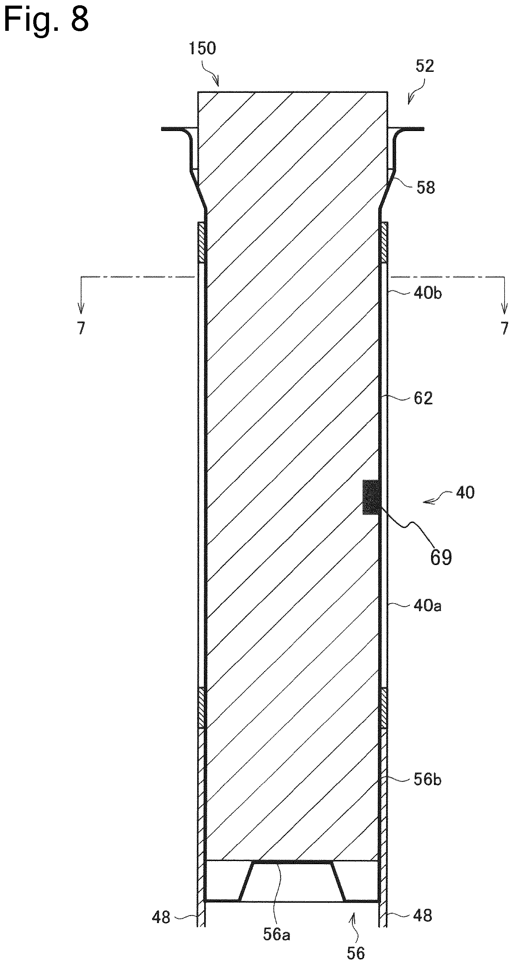

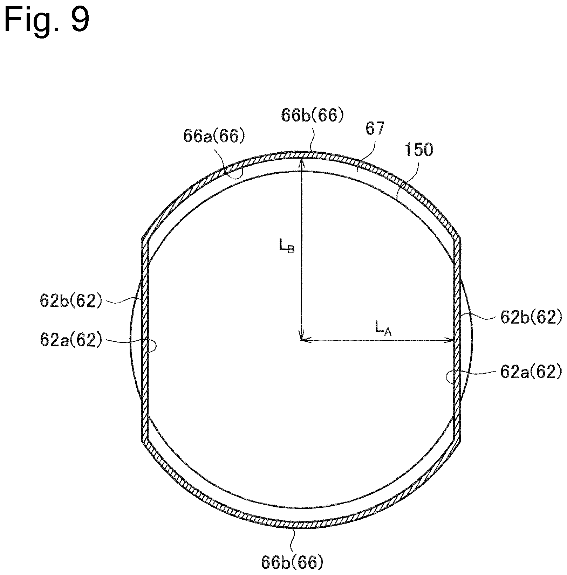

Fig. 7 is a longitudinal cross-section view of the chamber 50 which includes the non-pressing part 66 and is in the state that the stick-type base material 150 is being held in the holding part 60. Fig. 8 is a longitudinal cross-section view of the chamber 50 which includes the pressing part 62 and is in the state that the stick-type base material 150 is being held in the holding part 60. Fig. 9 is a cross-section view of the chamber 50, and relates to arrows 7-7 shown in Fig. 8. It should be reminded that the cross section of the stick-type base material 150 to which no pressure has been applied is shown in Fig. 9, to facilitate understanding of the process to apply pressure to the stick-type base material 150 by the pressing parts 62.

A space 67, which is shown in Fig. 9, positioned between the inner surface 66a of the non-pressing part 66 and the stick-type base material 150 is substantially maintained, even if the stick-type base material 150 is held in the holding part 60 and deformed as a result of application of pressure thereto by the pressing part 62. The space 67 can make the opening 52 of the chamber 50 and an the end surface, which is positioned in the chamber 50, of the stick-type base material 150 (the end surface shown in the lower center area in each of Fig. 7 and Fig. 8, i.e., the end surface of the base material 151) communicate with each other. In other words, the space 67 can make the opening 52 of the chamber 50 and an the end surface, that is positioned in the chamber 50 and is distant from the opening 52 of the chamber 50, of the stick-type base material 150 (the end surface shown in the lower center area in each of Fig. 7 and Fig. 8, i.e., the end surface of the base material 151) communicate with each other. Further, an air flow path from the opening 52 of the chamber 50 to an end surface, which is positioned in the outside of the chamber 50, of the stick-type base material 150 (the end surface shown in the upper center area in each of Fig. 7 and Fig. 8, i.e., the end surface of the suction opening part 52), which passes through the space 67 and the stick-type base material 150, is formed. By adopting the above construction, it becomes possible to simplify the construction of the inhaler 100, since it is not required to additionally construct a flow path for air, that is to be supplied to the stick-type base material 150, in the inhaler 100. Further, since a part, which forms a part of the space 67, in the non-pressing part 66 is exposed, cleaning of the flow path can be performed easily. Still further, since air is heated when it passes through the space 67, heat dissipated from the heater 40 is effectively used for increasing heating efficiency, and excessive falling of temperature of the stick-type base material 150 due to the air, that is taken in when puff action is performed, can be prevented. As a result, the quantity of consumption of electric power by the heater 40 can be reduced, and reducing of the flavor due to temperature falling of the stick-type base material 150 due to puff action can be prevented. In view of air-flow resistance and in view of other matters, the height of the space 67 between the inner surface 66a of the non-pressing part 66 and the stick-type base material 150 is set preferably to that equal to or greater than 0.1 mm and equal to or less than 1.0 mm; more preferably, the height is set to that equal to or greater than 0.2 mm and equal to or less than 0.8 mm; and, most preferably, the height is set to that equal to or greater than 0.3 mm and equal to or less than 0.5 mm.

As shown in Fig. 9, in the state that the stick-type base material 150 is being held in the holding part 60, the distance L_{A} between the inner surface 62a of the pressing part 62 and the center of the stick-type base material 150 is shorter than the distance L_{B} between the inner surface 66a of the non-pressing part 66 and the center of the stick-type base material 150. According to the above construction, the distance between the heater 40, that is positioned on the outer surface 62b of the pressing part 62, and the center of stick-type base material 150 can be shortened, compared with the distance in the case that the pressing part 62 is not formed. Thus, heating efficiency in the stick-type base material 150 can be increased.

As shown in Fig. 4 to Fig. 8, the chamber 50 comprises a bottom part 56. The bottom part 56 supports, by a bottom wall 56a, a part of the stick-type base material 150 inserted into the chamber 50 in such a manner that at least a part of the end surface of the stick-type base material 150 is exposed, as shown in Fig. 8. Further, the bottom part 56 may support, by the bottom wall 56a, a part of the stick-type base material 150 in such a manner that the exposed end surface of the stick-type base material 150 communicates with the space 67.

As shown in Fig. 5, Fig. 7, and Fig. 8, the bottom part 56 of the chamber 50 comprises the bottom wall 56a, and, in addition thereto, may comprise a side wall 56b. It may be possible to adopt a construction that the width, that depends on the side wall 56b, at a position in the bottom part 56 becomes smaller as the position approaches the bottom wall 56a closer. As shown in Fig. 6 and Fig. 9, the inner surface 66a of the non-pressing part 66 in the holding part 60 is curved, in a plane perpendicular to the longitudinal direction of the chamber 50.

It is preferable that, in arbitrarily selected positions in the longitudinal direction of the chamber 50, the shape of the inner surface 66a of the non-pressing part 66 in the holding part 60 in a plane perpendicular to the longitudinal direction of the chamber 50 be identical to the shape of the opening 52 in a plane perpendicular to the longitudinal direction of the chamber 50. In other words, it is preferable that the inner surface 66a of the non-pressing part 66 be constructed by extending, in the longitudinal direction, the inner surface of the chamber 50 which forms the opening 52.

As shown in Fig. 6 and Fig. 8, in the present embodiment, the holding part 60 is provided with a state detector 69 for detecting the state of the stick-type base material 150 held in the holding part 60. In more detail, the state detector 69 is arranged in a position on at least one surface of the pair of planar pressing surfaces in the inner surfaces 62a of the pressing parts 62, wherein the position is also that approximately in the center part in both the longitudinal and the lateral directions. For example, the state detector 69 may be constructed by using a pressure sensor to measure contact pressure applied by the stick-type base material 150. In this regard, instead of a position on the planar pressing surface, the state detector 69 may be arranged in any position that is to be in contact with a part of the held stick-type base material 150. For example, it may be arranged in a position in the bottom part 56 which supports a part of the stick-type base material 150. Further, it may be possible to provide plural state detectors 69.

As shown in Fig. 3 to Fig. 5, it is preferable that the chamber 50 be provided with a non-holding part 54 which has a cylindrical shape and is in a position between the opening 52 and the holding part 60. In the state that the stick-type base material 150 is being held in the holding part 60, a space may be formed in a position between the non-holding part 54 and the stick-type base material 150.

As shown in Fig. 5 to Fig. 9, it is preferable that the shape and the size of the outer peripheral surface of the holding part 60 (the length of the outer periphery of the holding part 60 in a plane perpendicular to the longitudinal direction of the holding part 60) be unchanged through the outer peripheral surface in the longitudinal direction of the holding part 60.

Further, as shown in Fig. 4 and Fig. 5, it is preferable that the chamber 50 be provided with a first guide part 58 which comprises a tapered surface 58a which is connected to the inner surface of the chamber 50, which forms the opening 52 and the inner surface 62a of the pressing part 62.

As shown in Fig. 3, the heater 40 comprises a heating element 42. The heating element 42 may be a heating track, for example. For example, as shown in Fig. 6, the outer surface 62b of the pressing part 62 and the outer surface 66b of the non-pressing part 66 are connected with each other with an angle between them, and a border 71 is formed in a position between the outer surface 62b of the pressing part 62 and the outer surface 66b of the non-pressing part 66. Preferably, the heating track extends in a direction that crosses a direction toward that the border 71 extends (the longitudinal direction of the chamber), and, preferably, extends in a direction perpendicular to the direction toward that the border 71 extends.

As shown in Fig. 3, it is preferable that the heater 40 be provided with, in addition to the heating element 42, an electrical insulation member 44 which covers at least one of surfaces of the heating element 42. In the present embodiment, the electrical insulation member 44 is arranged to cover the both surfaces of the heating element. Further, it is preferable that the electrical insulation member 44 be arranged in a region on the outer surface of the holding part 60. In other words, it is preferable that the electrical insulation member 44 be arranged in a region along the longitudinal direction of the chamber 50 and on the side of the first guide part 58 in such a manner that it does not protrude from the outer surface of the holding part 60. As explained above, since the first guide part 58 is arranged in the position between the opening 52 and the pressing part 62, the shape of the outer surface of the chamber 50 and the length of the outer periphery of the chamber in a plane perpendicular to the longitudinal direction of the chamber may change in the longitudinal direction of the chamber 50. Thus, the electrical insulation member 44 is arranged on the outer surface of the holding part 60, and, accordingly, slackening can be prevented.

Preferably, the heater 40 is not arranged on at least one of the outer surface of the chamber 50 positioned between the opening 52 and the first guide part 58, i.e., the outer surface of the non-holding part 54, the outer surface of the first guide part 58, and the outer surface of the non-pressing part 66. Preferably, the heater 40 is arranged on the overall outer surface 62b of the pressing part 62.

As shown in Fig. 3, the inhaler 100 comprises an electrode 48 that has a belt shape and extends from the heater 40. Preferably, in the state that the heater 40 is arranged on the outer surface 62b of the pressing opart 62, the belt-shaped electrode 48 extends toward the outside of the outer surface 62b, that is planar, of the pressing part 62 from the outer surface 62b of the pressing part 62.

Further, as shown in Fig. 3, Fig. 7, and Fig. 8, the heater 40 comprises a first part 40a which is in a position on a side opposite to the side of the opening 52, and a second part 40b which is in a position on the side of the opening 52. It is preferable that the heater power density in the second part 40b be higher than the heater power density in the first part 40a. Alternatively, it is preferable that the rate of temperature increase in the second part 40b be higher than the rate of temperature increase in the first part 40a. Alternatively, it is preferable that, at a given time, the heating temperature in the second part 40b be higher than the heating temperature in the first part 40a. Preferably, in the state that the stick-type base material 150 is being held in the holding part 60, the second part 40b covers the outer surface of holding part 60 in such a manner that the outer surface corresponding to a half or more than a half, in a longitudinal direction of a smokable article included in the stick-type base material 150, of the smokable article is covered.

In the embodiment explained above, the chamber 50 comprises a pair of pressing parts 62 which face each other; however, the shape of the chamber is not limited to that explained above. For example, the chamber 50 may comprise a single pressing part 62, or may comprise three or more pressing parts 62.

As explained above, the inhaler 100 holds the stick-type base material 150 by applying pressure thereto by the pressing part 62, and heats the stick-type base material 150. By adopting the above construction, it becomes possible to obtain various kinds of effect that are explained below.

First, the thermal conductivity from the heater 40 to the stick-type base material 150 increases. Thus, the heating efficiency with respect to the stick-type base material 150 is improved. Since the heating efficiency with respect to the stick-type base material 150 is improved, the temperature of the stick-type base material 150 can be increased to target temperature quickly; accordingly, the length of time required for preheating, that will be explained later, can be shortened. Further, since the heating efficiency with respect to the stick-type base material 150 is improved, temperature followability of the stick-type base material 150 relating to temperature change in the heater 40 can be improved. As a result, first, controlling of the quantity of aerosol, that is to be generated, is further facilitated. Second, even if the temperature of the stick-type base material 150 is lowered in relation to puff action performed by a user, the lowered temperature can be increased quickly to the original temperature. Third, effect of external environment, such as ambient temperature and so on, thereon can be reduced. Fourth, temperature change that is similar to temperature change defined in a heating profile, that will be explained later, can be realized easily in the stick-type base material 150. Fifth, flavor enhancing effect, that is the effect given during a temperature re-increasing section in a heating profile that will be explained later, can be provided quickly.

Further, the inhaler 100 heats the stick-type base material 150 from the outer periphery thereof while applying pressure thereto. According to the above construction, improvement of heating efficiency with respect to the stick-type base material 150 and improvement of followability with respect to temperature of the stick-type base material 150, that has been explained above, can be realized, independent of the shape of the aerosol source in the stick-type base material 150. Further, according to the above construction, improvement of heating efficiency with respect to the stick-type base material 150 and improvement of followability with respect to temperature of the stick-type base material 150, that has been explained above, can be realized, independent of errors with respect to the shape and the size of the stick-type base materials 150 that are included due to variation of the stick-type base materials 150 that occurs during the manufacturing process thereof. On the other hand, in a comparative example which comprises a construction that a blade-shaped heater 40 is inserted into a stick-type base material 150 and the stick-type base material 150 is heated from the inside thereof, it is difficult to provide effects such as those explained above. This is because, in the above comparative example, even if pressure is applied to the stick-type base material 150 from the outer periphery, it is difficult to successfully bring the blade-shaped heater 40 and the aerosol source in the stick-type base material 150 into contact with each other.

Further, in the inhaler 100, the heat insulator 70 is arranged to surround the heater 40 from the side of the outer periphery thereof. In the above case, since the outer surface 62b of the pressing part 62 arranged in a position is closer to the center of the inner space 80 than the position of the outer surface 66b of the non-pressing part 66 when the positions are compared with each other, the thickness of an air layer formed in a position between the outer surface 62b of the pressing part 62 and the heat insulator 70 can be made large. Alternatively, the thickness of the heat insulator 70 positioned on the pressing part 62 can be made large. Thus, heat insulating effect given by the heat insulator 70 can be improved.

### « 2. Technical Characteristics »

### (2-1) Control of Heating Operation Based on Heating Profile

The inhaler 100 controls, based on a heating profile, operation of the heater 40 (i.e., heating operation). In more detail, for realizing chronological change of target temperature that has been defined in a heating profile, the inhaler 100 controls temperature of the heater 40 by controlling operation of the electric power source 111 for supplying electric power to the heater 40. The heating profile is an example of information that defines chronological change of target temperature that corresponds to target values of temperature of the heater 40. The heating profile is stored in the memory 114. The heating profile is referred to when heating operation of the heater 40 is performed for heating the stick-type base material 150. Further, instead of simply referring to the heating profile, it may be possible to change the heating profile during operation for heating the stick-type base material 150.

By adopting the above construction, aerosol is generated in accordance with a plan defined in the heating profile, in response to operation of the heater 40 in accordance with the heating profile. Typically, the heating profile is designed in such a manner that the flavor that a user tastes becomes the optimum state thereof when the user has performed an inhalation action. Thus, by controlling, based on the heating profile, operation for supplying electric power from the electric power source 111 to the heater 40, the state of the flavor that a user tastes can be made optimum.

The controller 116 controls operation of the heater 40 based on a difference (separation) between target temperature defined in the heating profile and actual temperature of the heater 40 (hereinafter, this may be referred to as "actual temperature"). In more detail, the controller 116 controls operation of the electric power source 111 for supplying electric power to the heater 40, based on a separation between target temperature of the heater, that corresponds to time elapsed since the time of a start of control based on the heating profile for supplying electric power to the heater 40, and actual temperature of the heater 40. The controller 116 controls the temperature of the heater 40 in such a manner that chronological change of actual temperature of the heater 40 becomes the same as chronological change of target temperature of the heater 40 defined in the heating profile. The temperature control of the heater 40 can be realized by using publicly-known feedback control, for example. Specifically, the controller 116 makes the electric power source 111 supply, to the heater 40, electric power in the forms of pulses generated by performing pulse width modulation (PWM) or pulse frequency modulation (PFM). In the above case, the controller 116 can perform temperature control of the heater 40 by adjusting the duty ratio of the electric power pulse.

In the feedback control, the controller 116 may control, based on difference between the actual temperature and the target temperature or the like, electric power supplied from the electric power source 111 to the heater, for example, the above-explained duty ratio. The feedback control may be PID control (a Proportional-Integral-Differential Controller), for example. In a different construction, the controller 116 may perform simple ON-OFF control. For example, the controller 116 may instruct the electric power source 111 to make the heater 40 perform heating operation until actual temperature reaches target temperature, make the heater 40 stop heating operation in the case that actual temperature has reached target temperature, and make the heater 40 perform heating operation again when actual temperature has become that below target temperature.

The temperature of the heater 40 can be determined, for example, by measuring or estimating the value of electric resistance of a heat-generating resistor which is included in the heater 40. This is because the electric resistance value of the heat-generating resistor changes according to temperature. The electric resistance value of the heat-generating resistor may be estimated by measuring the quantity of a voltage drop in the heat-generating resistor, for example. The quantity of the voltage drop in the heat-generating resistor can be measured by a voltage sensor for measuring a potential difference applied to the heat-generating resistor. In a different example, the temperature of the heater 40 may be measured by using a temperature sensor arranged in a position close to the heater 40.

The measured temperature of the heater 40 may be used in judgment with respect to puff action performed by a user.

Heating based on the heating profile is started at timing when completion of manipulation representing an instructing for starting heating is detected. An example of manipulation representing an instructing for starting heating is pressing of a button constructed in the inhaler 100. The other example of manipulation representing an instructing for starting heating is a puff action. The other example of manipulation representing an instructing for starting heating is reception of a signal from the other device such as a smartphone or the like.

After starting of heating, the quantity of the aerosol source included in the base material gradually decreases as time elapses. Typically, at timing when the aerosol source is expected to be exhausted, heating operation performed by the heater 40 is stopped. An example of timing when the aerosol source is expected to be exhausted is timing when a predetermined period of time has elapsed since a start of controlling of operation of the heater 40 based on the heating profile. An example of timing when the aerosol source is expected to be exhausted is timing when a predetermined number of times of puffs is detected. An example of timing when the aerosol source is expected to be exhausted is timing when a button constructed in the inhaler 100 is pressed. For example, the above button is pressed when a user has felt that the user cannot taste flavor satisfactory.

In this regard, a period during that a sufficient quantity of aerosol is expected to be generated is also referred to as a puff allowable period. On the other hand, a period from a point in time when heating is started to a point in time when a puff allowable period starts is also referred to as a preheating period. Heating performed during the preheating period is also referred to as preheating. A user may be notified of timing when the puff allowable period starts and timing when it ends. In the above case, a user can use above notification for reference, and, based thereon, can perform puff action during the puff allowable period.

The controller 116 controls operation of the heater 40, based on a holding state of the stick-type base material 150 held by the holding part 60. In more detail, the controller 116 controls operation of the heater 40 to heat the stick-type base material 150 based on the heating profile, in the state that a part of the stick-type base material 150 is being pressed by the pressing part 62 in the holding part 60. That is, the controller 116 controls heating of the stick-type base material 150 by the heater 40 in the state that a part of the stick-type base material 150 is being pressed by the pressing part 62 in the holding part 60, by adjusting the quantity of electric power supplied to the heater 40 in relation to target temperature corresponding to the length of time elapsed since the time when control of operation of the heater 40 based on the heating profile is started. In the above process, the controller 116 may further adjust the quantity of supplied electric power in relation to the strength of pressure applied by the pressing part 62. Further, the controller 116 may control operation of the heater 40 to prevent heating of the stick-type base material 150 based on the heating profile (for example, supplying of electric power to the heater 40 is stopped), in the state that no part of the stick-type base material 150 is pressed by the pressing part 62 in the holding part 60. According to the above construction, it becomes possible to control operation of the heater 40 in relation to the degree of increase in the heating efficiency with respect to the stick-type base material 150, when the matter that the heating efficiency with respect to the stick-type base material 150 is increased by pressing it is taken into consideration. Thus, it becomes possible to provide a user with quality puff experience.

### (2-2) Basic Mode of Temperature Change

The heating profile comprises plural consecutive time sections along a time axis. Each of the plural time sections is set in such a manner that it is associated with target temperature that is expected to be reached in an end stage in the time section. Thus, the controller 116 controls operation of the heater 40, based on a separation between actual temperature and target temperature relating to a time section, in the plural time sections, that corresponds to a period of time elapsed since controlling of operation of the heater 40 based on the heating profile is started. Specifically, the controller 116 controls operation of the heater 40 in such a manner that the actual temperature reaches target temperature, that has been set to be related to each of the plural time sections included in the heating profile, before an end stage in the time section ends. An example of the heating profile is shown in Table 1 below.

**(Table 1)**

| Table 1. Example of Heating Profile | | |
|---|---|---|
| Time Section | Length of Time | Target Temperature |
| Initial Temperature-Increasing Section | 35 seconds | 295 °C |
| Middle Temperature-Decreasing Section | 10 seconds | 230 °C |
| Temperature Re-increasing Section | 310 seconds | 260 °C |

The heating profile shown in Table 1 comprises an initial temperature-increasing section, a middle temperature-decreasing section, and a temperature re-increasing section in this order. In the example shown in Table 1, the initial temperature-increasing section is a section from a point in time when the heating profile is started to a point in time when 35 seconds has elapsed since the start of the heating profile. The middle temperature-decreasing section is a section from a point in time when the initial temperature-increasing section ends to a point in time when 10 seconds has elapsed since the end of the initial temperature-increasing section. The temperature re-increasing section is a section from a point in time when the middle temperature-decreasing section ends to a point in time when 310 seconds has elapsed since the end of the middle temperature-decreasing section. As a result that the above time sections are included in the heating profile, it becomes possible to provide a user with quality puff experience through the period from a start to an end of the heating profile, as will be explained below. That is, it becomes possible to improve the quality of puff experience that a user is provided with.

The initial temperature-increasing section is the time section included in a first part of the heating profile. The value of the target temperature that has been set to be related to the initial temperature-increasing section is higher than an initial value. The initial value is a value corresponds to temperature that is assumed as that of the heater 40 before starting of heating. An example of the initial value is 0 degree Celsius or the like, and the initial value can be selected arbitrarily. The other example of the initial value is a value corresponding to atmospheric temperature.

The middle temperature-decreasing section is the time section included in the middle part of the heating profile. The target temperature that has been set to be related to the middle temperature-decreasing section is lower than the target temperature that has been set to be related to the time section just before the middle temperature-decreasing section. In the example shown in Table 1, the target temperature, specifically, 230 degrees Celsius, that has been set in relation to the middle temperature-decreasing section is lower than the target temperature, specifically, 295 degrees Celsius, that has been set in relation the initial temperature-increasing section that is the time section just before the middle temperature-decreasing section.

The temperature re-increasing section is the time section included in the end part of the heating profile. The target temperature that has been set to be related to the temperature re-increasing section is higher than the target temperature that has been set to be related to the time section just before the temperature re-increasing section. In the example shown in Table 1, the target temperature, specifically, 260 degrees Celsius, that has been set in relation to the temperature re-increasing section is higher than the target temperature, specifically, 230 degrees Celsius, that has been set in relation the middle temperature-decreasing section that is the time section just before the temperature re-increasing section.

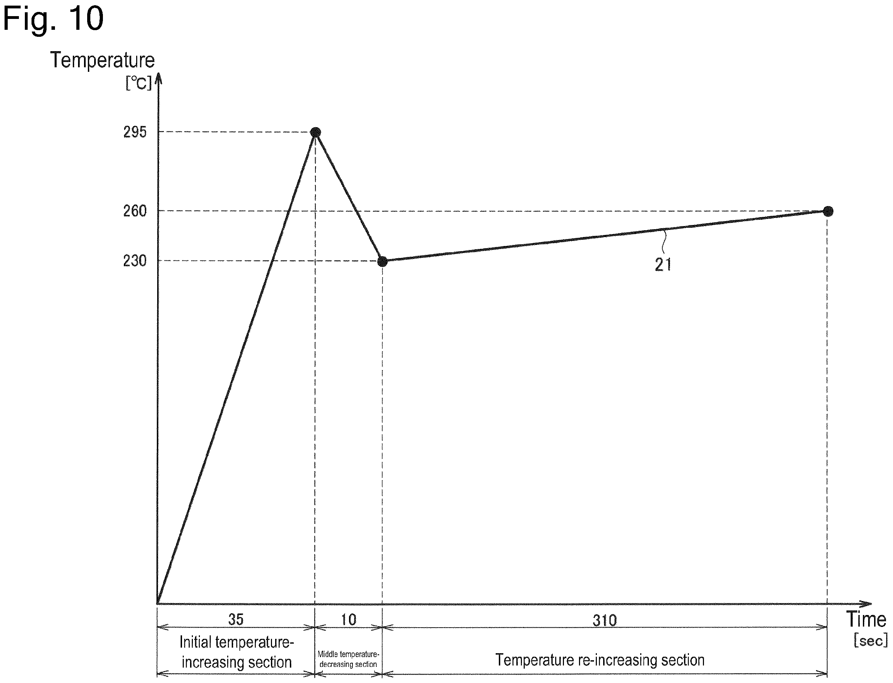

Chronological change in actual temperature of the heater 40, in the case that operation of the heater 40 is controlled by the controller 116 according to the heating profile shown in Table 1, will be explained with reference to Fig. 10. Fig. 10 is a graph which shows an example of chronological change in actual temperature of the heater 40 which was operated based on the heating profile shown in Table 1. The horizontal axis of the graph represents time (seconds). The vertical axis of the graph represents temperature of the heater 40. The line 21 in the graph represents chronological change in actual temperature of the heater 40.

As shown in Fig. 10, the actual temperature of the heater 40 has increased in the initial temperature-increasing section, and has reached 295 degrees Celsius, that is the target temperature, in the end stage in the initial temperature-increasing section. In the case that the actual temperature of the heater 40 has reached the target temperature that has been set in relation to the initial temperature-increasing section, it can be assumed that the temperature of the stick-type base material 150 reaches temperature that that makes it possible to generate a sufficient quantity of aerosol. The initial temperature-increasing section is set to be arranged in a first part in the heating profile. Thus, in the initial temperature-increasing section, the heater 40 is heated to increase the temperature immediately from initial temperature to 295 degrees Celsius that is target temperature that has been set in relation to the initial temperature-increasing section. In this regard, the initial temperature is actual temperature of the heater 40 at the time when heating based on the heating profile is started. According to the above construction, it becomes possible to terminate preheating in an early stage.

The controller 116 performs, during the initial temperature-increasing section, temperature control of the heater 40 to change actual temperature to make it reach target temperature that has been set in relation to the initial temperature-increasing section. That is, the controller 116 controls temperature of the heater 40 to make it reach 295 degrees Celsius. If the actual temperature has reached 295 degrees Celsius before the elapse of 35 seconds from the start of heating, the controller 116 controls the temperature of the heater 40 to keep it at 295 degrees Celsius.

As shown in Fig. 10, the actual temperature of the heater 40 has decreased in the middle temperature-decreasing section, and has reached 230 degrees Celsius, that is target temperature, in an end stage in the middle temperature-decreasing section. The middle temperature-decreasing section is set to be arranged to follow the initial temperature-increasing section. Thus, in the middle temperature-decreasing section, the controller 116 makes the temperature drop temporarily from the set temperature relating to the initial temperature-increasing section to the set temperature relating to the middle temperature-decreasing section. If the temperature of the heater 40 is maintained at high temperature such as the target temperature relating to the initial temperature-increasing section, the aerosol source included in the stick-type base material 150 may be consumed rapidly, and disadvantageous matters such that the flavor that is to be tasted by a user becomes excessively strong, and so on, may occur. That is, by adding the middle temperature-decreasing section, it becomes possible to avoid the above disadvantageous matters and improve the quality of puff experience of a user.

In the middle temperature-decreasing section, the controller 116 performs control to stop supplying of electric power to the heater 40. That is, in the middle temperature-decreasing section, the controller 116 performs control to stop supplying of electric power to the heater 40 to prevent heating performed by the heater 40. By using the above construction, the actual temperature of the heater 40 can be lowered at the fastest speed. Further, it becomes possible to reduce electric consumption of the inhaler 100, compared with that in a case wherein supplying of electric power to the heater 40 is performed during the middle temperature-decreasing section.

As shown in Fig. 10, the actual temperature of the heater 40 has increased during the temperature re-increasing section, and reached 260 degrees Celsius, that is target temperature, in an end stage in the temperature re-increasing section. The temperature re-increasing section is set to be arranged in a last part in the heating profile and follows the middle temperature-decreasing section. Thus, in the temperature re-increasing section, the heater 40 is heated again to increase the temperature from the set temperature relating to the middle temperature-decreasing section to the set temperature relating to the temperature re-increasing section. If the heater 40 is controlled to make its temperature decrease continuously after completion of the initial temperature-increasing section, the temperature of the stick-type base material 150 also decreases; accordingly, the quantity of generated aerosol decreases, and the flavor that is to be tasted by a user may be deteriorated thereby. That is, by adding the temperature re-increasing section that follows the middle temperature-decreasing section, it also becomes possible to prevent deterioration of flavor, that is to be tasted by a user, in the latter half of the heating profile.

The controller 116 performs, during the temperature re-increasing section, temperature control of the heater 40 to change actual temperature to make it reach target temperature that has been set in relation to the temperature re-increasing section. That is, the controller 116 controls temperature of the heater 40 to make it reach 260 degrees Celsius. If the actual temperature has reached 260 degrees Celsius before the elapse of 310 seconds from the start of the temperature re-increasing section, the controller 116 controls the temperature of the heater 40 to keep it at 260 degrees Celsius.

When the absolute vale of the quantity of change in target temperature per unit time with respect to each of the initial temperature-increasing section, the middle temperature-decreasing section, and the temperature re-increasing section is compared with others, it can be understood that the absolute vale with respect to the temperature re-increasing section may be the smallest, the absolute vale with respect to the middle temperature-decreasing section may be the next smallest, and the absolute vale with respect to the initial temperature-increasing section may be the largest among them. The absolute vale of the quantity of change in target temperature per unit time with respect to the initial temperature-increasing section is a value obtained by dividing, by the time length of the initial temperature-increasing section, the absolute value of the difference between the initial value and the value of the target temperature that has been set in relation to the initial temperature-increasing section. The absolute vale of the quantity of change in target temperature per unit time with respect to the middle temperature-decreasing section is a value obtained by dividing, by the time length of the middle temperature-decreasing section, the absolute value of the difference between the target temperature that has been set in relation to the middle temperature-decreasing section and the target temperature that has been set in relation to a time section just before the middle temperature-decreasing section (for example, the initial temperature-increasing section). The absolute vale of the quantity of change in target temperature per unit time with respect to the temperature re-increasing section is a value obtained by dividing, by the time length of the temperature re-increasing section, the absolute value of the difference between the target temperature that has been set in relation to the temperature re-increasing section and the target temperature that has been set in relation to a time section just before the temperature re-increasing section (for example, the middle temperature-decreasing section). Further, when the length of time of each of the initial temperature-increasing section, the middle temperature-decreasing section, and the temperature re-increasing section is compared with others, it can be understood that the middle temperature-decreasing section is the shortest, the initial temperature-increasing section is the next shortest, and the temperature re-increasing section is the longest among them. As shown in Fig. 10, according to the above construction, the temperature of the heater 40 is rapidly increased in the initial temperature-increasing section, the heater 40 exits early from the high temperature state in the middle temperature-decreasing section, and the temperature of the heater 40 is slowly increased in the temperature re-increasing section. Thus, it becomes possible to complete preheating at an early stage, and provide a user with quality puff experience though the beginning to the end of the heating profile.

The controller 116 may perform, based on the actual temperature of the heater 40, determination with respect a part of switching operation for switching between plural time sections in the heating profile. For example, the controller 116 may perform determination with respect to switching from the initial temperature-increasing section to the middle temperature-decreasing section, and terminating of the temperature re-increasing section, based on whether a separation between the target temperature, that has been set in relation to each related time section, and the actual temperature of the heater 40 has become that in a predetermined threshold value range.

The controller 116 may perform, based on time elapsed, determination with respect a part of switching operation for switching between plural time sections in the heating profile. For example, the controller 116 may determine the end of the middle temperature-decreasing section, based on the time elapsed since a start of the middle temperature-decreasing section. For example, in the heating profile shown in Fig. 10, the length of the middle temperature-decreasing section is set to 10 seconds. Thus, the controller 116 determines switching to the temperature re-increasing section when 10 seconds has elapsed since a start of the middle temperature-decreasing section, and makes the heater 40 restart heating. According to the above construction, switching from the middle temperature-decreasing section to the temperature re-increasing section can be performed without measuring temperature of the heater 40; thus, the processing load on the controller 116 can be reduced. Further, even in the case that a construction for measuring temperature of the heater 40 based on an electric resistance value of a heat-generating resistor included in the heater 40 is adopted, it becomes possible to determine switching to the temperature re-increasing section, while stopping supplying of electric power to the heater 40 in the middle temperature-decreasing section.

It should be reminded that the actual temperature of the heater 40 in the end stage of the middle temperature-decreasing section may change in relation to external environment, such as ambient temperature and so on. For example, in the case that operation is performed based on the heating profile shown in Fig. 10, the actual temperature of the heater 40 in the end stage of the middle temperature-decreasing section may become 220 degrees Celsius when ambient temperature is low, and may become 240 degrees Celsius when ambient temperature is high.

Thus, in a first stage in the next time section following the middle temperature-decreasing section (that is, the temperature re-increasing section), operation of the heater 40 is controlled based on the actual temperature of the heater 40 and the target temperature that has been set in relation to the middle temperature-decreasing section. In more detail, the controller 116 performs operation for supplying electric power to the heater 40 at a first duty ratio, in the first stage in the next time section following the middle temperature-decreasing section, if the actual temperature of the heater 40 is less than the target temperature that has been set in relation to the middle temperature-decreasing section. On the other hand, the controller 116 performs operation for supplying electric power to the heater 40 at a second duty ratio, in the first stage in the next time section following the middle temperature-decreasing section, if the actual temperature of the heater 40 is equal to or higher than the target temperature that has been set in relation to the middle temperature-decreasing section. In the present case, the first duty ratio is higher than the second duty ratio. The duty ratio in the present case refers to a ratio of a period of time during that electric power is supplied to the heater 40 in a predetermined period. According to the above construction, even in the case that a separation between the target temperature and the actual temperature of the heater 40 has occurred due to effect of external environment, the separation can be reduced quickly, so that deterioration of flavor that is to be tasted by a user can be suppressed.

### (2-3) Flow of Processing Relating to Control of Heating Operation

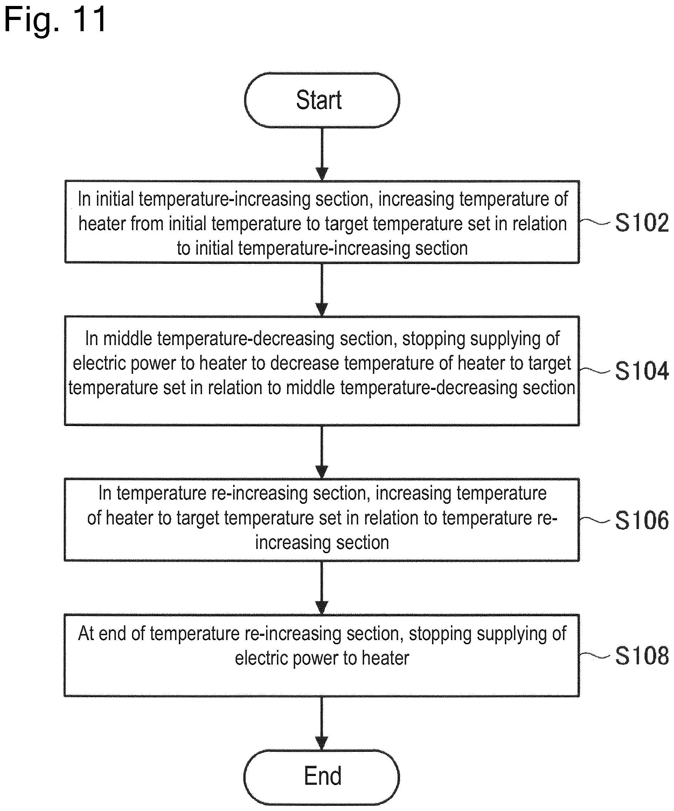

Fig. 11 is a flow chart showing an example of the flow of processing performed by the inhaler 100. First, the inhaler 100 performs operation for increasing, in the initial temperature-increasing section, the temperature of the heater 40 from initial temperature to the target temperature that has been set in relation to the initial temperature-increasing section (step S102). Next, the inhaler 100 performs, in the middle temperature-decreasing section, operation for stopping supplying of electric power to the heater 40 to decrease the temperature of the heater 40 to the target temperature that has been set in relation to the middle temperature-decreasing section (step S104). Next, the inhaler 100 performs operation for increasing, in the temperature re-increasing section, the temperature of the heater 40 to the target temperature that has been set in relation to the temperature re-increasing section (step S 106). Thereafter, when the temperature re-increasing section ends, the inhaler 100 performs operation for stopping supplying of electric power to the heater 40 (step S108).

### (2-4) Modified Mode of Temperature Change

Each of the initial temperature-increasing section, the middle temperature-decreasing section, and the temperature re-increasing section may comprise a temperature maintaining section for maintaining the temperature of the heater 40 at fixed temperature. An example of a heating profile in the above case is shown in Table 2.

**(Table 2)**

| Table 2. Example of Heating Profile | | | |
|---|---|---|---|
| Time Section | | Length of Time | Target Temperature |
| Initial Temperature-Increasing Section | Temperature Increasing Section | 25 seconds | 295 °C |
| | Temperature Maintaining Section | 10 seconds | 295 °C |
| Middle Temperature-Decreasing Section | Temperature Decreasing Section | 10 seconds | 230 °C |
| | Temperature Maintaining Section | 135 seconds | 230 °C |
| Temperature Re-increasing Section | Temperature Increasing Section | 80 seconds | 260 °C |
| | Temperature Maintaining Section | 140 seconds | 260 °C |

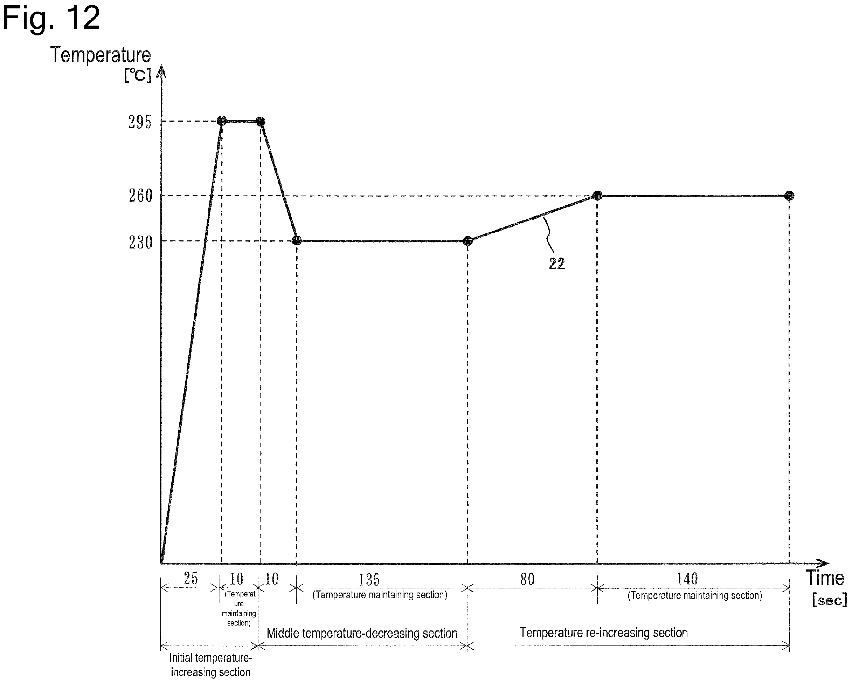

Fig. 12 is a graph which shows an example of chronological change in actual temperature of the heater 40 which was operated based on the example heat profile shown in Table 2. The horizontal axis of the graph represents time (seconds). The vertical axis of the graph represents temperature (degrees Celsius) of the heater 40. The line 22 in the graph represents chronological change in actual temperature of the heater 40.

In the example shown in Table 2, the initial temperature-increasing section is a section from a point in time when the heating profile starts to a point in time when 35 seconds has elapsed since the above start. The period of first 25 seconds in the initial temperature-increasing section is a temperature increasing section, and forms a time section for preheating for initially increasing the target temperature from 0 degree Celsius to 295 degrees Celsius. Further, the period of last 10 seconds (that follows the temperature increasing section) is a temperature maintaining section for maintaining the target temperature at 295 degrees Celsius.

The middle temperature-decreasing section, that follows the initial temperature-increasing section, is a section from a point in time when the initial temperature-increasing section ends to a point in time when 145 seconds has elapsed since the above end. The period of first 10 seconds in the middle temperature-decreasing section is a temperature decreasing section, and the target temperature is decreased from 295 degrees Celsius to 230 degrees Celsius therein. Further, the period of last 135 seconds (that follows the temperature decreasing section) is a temperature maintaining section for maintaining the target temperature at 230 degrees Celsius.

The temperature re-increasing section, that follows the middle temperature-decreasing section, is a section from a point in time when the middle temperature-decreasing section ends to a point in time when 220 seconds has elapsed since the above end. The period of first 80 seconds in the temperature re-increasing section is a temperature increasing section, and the target temperature is again increased from 230 degrees Celsius to 260 degrees Celsius therein. Further, the period of last 140 seconds (that follows the temperature increasing section) is a temperature maintaining section for maintaining the target temperature at 260 degrees Celsius.

In the example shown in Table 2, the target temperature in the initial temperature-increasing section is 295 degrees Celsius, the target temperature in the middle temperature-decreasing section is 230 degrees Celsius, and the target temperature in the temperature re-increasing section is 260 degrees Celsius, and the above temperatures constitute target temperatures (hereinafter, section's target temperatures) that are related to the above time sections, respectively. The section's target temperatures are defined in such a manner that they relate to time sections in the heating profile, respectively.

In this regard, one or plural values with respect to the section's target temperature/temperatures may be set. Further, it is not necessarily required to set target temperature in a first stage and target temperature in a last stage in a temperature maintaining section to have an identical value.

As a result that the heating profile is constructed to include the above time sections, it becomes possible to provide a user with quality puff experience through the heating profile, i.e., from the start to the end of the heating profile. Especially, by adopting the construction that the temperature maintaining section is included in each of the initial temperature-increasing section, the middle temperature-decreasing section, and the temperature re-increasing section, it becomes possible to effectively increase, in the initial temperature-increasing section and the temperature re-increasing section, the temperature of the stick-type base material 150 including that of the inside part thereof. Further, it becomes possible to sufficiently decrease the temperature of the stick-type base material 150 including that of the inside part thereof, in the middle temperature-decreasing section. That is, it becomes possible to improve the quality of user's puff experience.

### (2-5) Construction Example Relating to Judgment with Respect to Puff Action

In the following description, operation for detecting puff action, that is performed by a user by using the inhaler 100 according to the present embodiment, will be explained in detail.

The controller 116 in the inhaler 100 has a function for detecting puff action performed by a user, and controlling, based on the number of times of puff actions, heating operation with respect to the stick-type base material 150. For example, the controller 116 in the inhaler 100 can perform operation for terminating heating operation, in response to detection of a predetermined number of times of puff actions.

As explained above, when the stick-type base material 150 is held in a user's mouth and an inhalation action is performed by the user, the air flows through the opening 52 into the space between the heater assembly 30 (which includes the heater 40) and the stick-type base material 150. The inflow air cools the heater 40, so that the temperature thereof is lowered. By using cooling effect such as that explained above, change in the quantity of air entering the inhaler 100 may be detected. That is, in an example, for detecting puff action performed by a user, it may be possible to consider adoption of a construction for detecting change in temperature of the heater 40 that occurs in relation to puff action performed by a user.

In this regard, change in temperature of a heating element can be detected by using any of various kinds of methods. For example, change in temperature of a heating element can be detected by arranging a temperature sensor (thermistor) in a position close to the heating element. Also, instead of using a thermistor, change in temperature of a heating element can be estimated by using the quantity of change in the electric resistance value of a heat-generating resistor, the quantity of change in the quantity of electric power supplied to the heating element, or the like.

On the other hand, in general, it has been known that the quantity of change in detected temperature, that is observed in relation to a series of puff actions, may not be stable always. That is, in the method for detecting puff action which adopts a process for detecting change in temperature, there is room for improvement in view of accuracy in terms of detection. Specifically, it has been know experimentally that there is a tendency that the variation width regarding the quantity of temperature change relating to a series of puff actions becomes smaller as the time elapsed since a start of heating by the heating element becomes longer. That is, in the case that detection of change in temperature is performed by using a thermistor, accuracy in terms of detection may become more unstable, as the operation process relating to the heating period proceeds closer to the end stage in the heating period and the variation width regarding the quantity of temperature change becomes smaller accordingly. The above matter is also true in the case that change in temperature is detected by using a method that uses the quantity of change in the electric resistance value of a heat-generating resistor or the quantity of change in the value of the electric power supplied to the heating element. Further, in the above case, since it is necessary to additionally install a sensor for measuring the electric resistance value or the like, the cost and the size of the device may be increased due thereto.

Thus, a further object of the inhaler 100 according to the present embodiment is to realize, by using a simple method, a method for accurately judging whether a puff action is performed by a user.

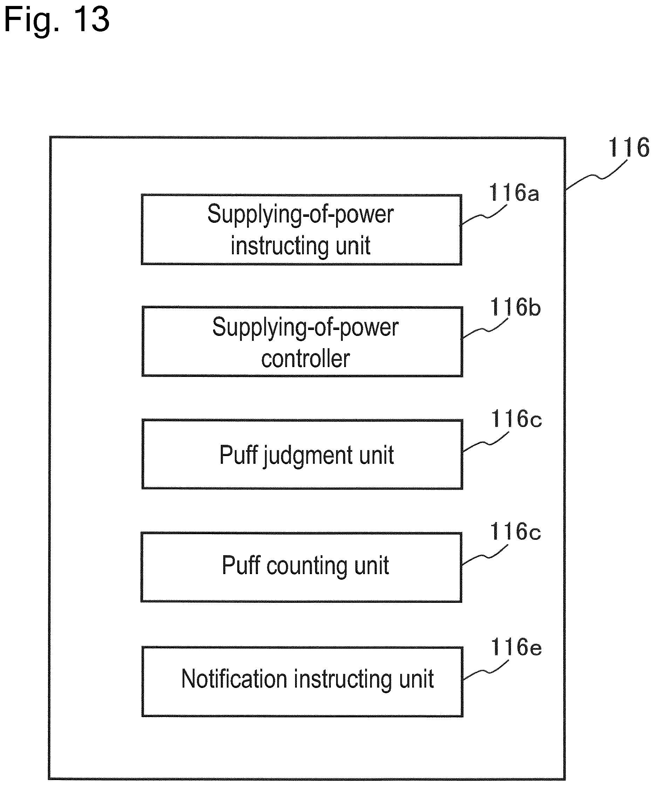

Fig. 13 is a block diagram which functionally shows a construction of the controller 116 which performs a control method according to the present embodiment. The controller 116 included in the inhaler 100 comprises a supplying-of-power instructing unit 116a, a supplying-of-power controller 116b, a puff judgment unit 116c, a puff counting unit 116d, and a notification instructing unit 116e.

The supplying-of-power instructing unit 116a instructs the electric power source 111 to start or stop supplying of electric power to the heater 40.

The supplying-of-power controller 116b controls operation of the electric power source 111 for supplying electric power to the heater 40, to change the temperature to make it reach target temperature defined in the heating profile. As explained above, the target temperature defined in the heating profile may be set in such a manner that target temperatures are set to correspond to time sections, respectively, wherein the respective time sections correspond to the respective lengths of time elapsed since a start of operation for supplying electric power. Examples of the time sections are the initial temperature-increasing section, the middle temperature-decreasing section, and the temperature re-increasing section that are shown in Fig. 10 and Fig. 12. That is, the supplying-of-power controller 116b performs operation for adjusting the temperature of the heater 40 to make the temperature of the heater 40 reach target temperature in an end stage in a corresponding time section. Adjusting of the temperature of the heater 40 may be realized by using the above-explained publicly known feedback control.

The puff judgment unit 116c performs judgment as to whether a control value related to operation for supplying electric power satisfies a predetermined condition relating to change. In the case that the control value satisfies the predetermined change condition, it is judged that puff action is performed by a user. The control value is a value used by the supplying-of-power controller 116b in controlling of operation for supplying electric power, and, in the above-explained example, the control value may be a duty ratio of a pulse of electric power. Further, the change condition with respect to the control value may be set as a condition that defines that the quantity of increase in the duty ratio is greater than a predetermined threshold value. That is, the puff judgment unit 116c judges that a puff action has been performed, in the case that the quantity of increase in the duty ratio used in controlling of operation for supplying electric power has become larger than a predetermined threshold value.

The puff counting unit 116d counts, in heating control based on the heating profile, the number of puff actions performed by a user.

The notification instructing unit 116e instructs the notifier 113 to perform predetermined notification operation. For example, in response to a judgment that is made by the puff judgment unit 116c and indicates that a puff action has been performed by a user, the notification instructing unit 116e makes the notifier 113 perform notification operation for informing the above matter.

### (2-6) Example of Judgment with Respect to Puff Action

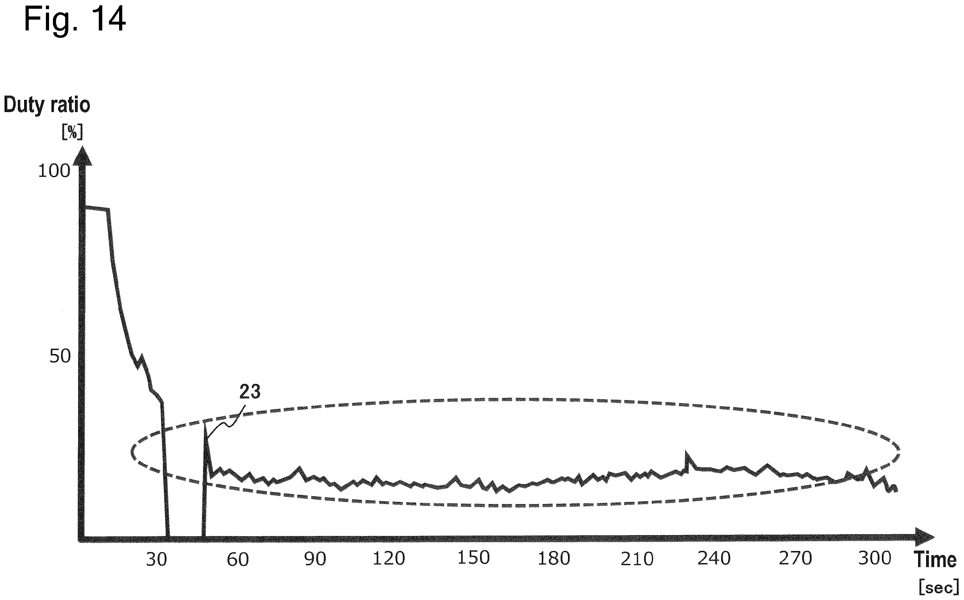

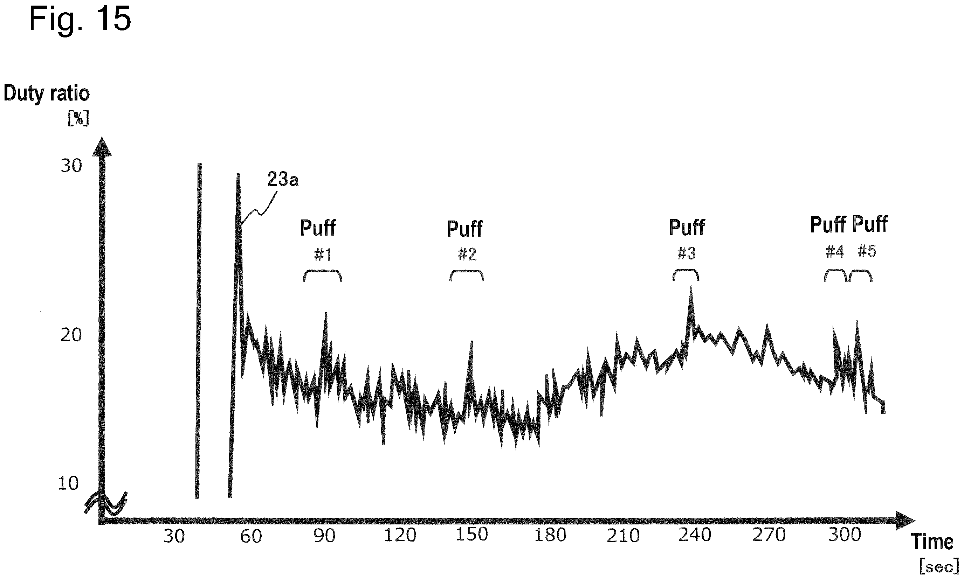

Each of Fig. 14 and Fig. 15 is a graph which shows an example of chronological change in the duty ratio in controlling of operation for supplying electric power. The graph shown in Fig. 14 represents the overall chronological change in the duty ratio, and an enlarged graph that corresponds to a part encircled by a broken line in Fig. 14 is shown in Fig. 15. In each graph, the horizontal axis represents time (seconds), and the vertical axis represents the duty ratio (%) of the electric power pulse. In this regard, each graph corresponds to the heating profile shown in Fig. 10.

As shown by the line 23 in the graph in Fig. 14, the supplying-of-power controller 116b controls operation for supplying electric power, by determining the duty ratio in real time by using feedback control. As explained above, in the case that the stick-type base material 150 is held in a user's mouth and inhalation action is performed by the user, the air flowing through the opening 52 cools the heater 40. As a result, the temperature of the heater 40 decreases. In response thereto, supplying-of-power controller 116b makes compensation for the decrease in temperature of the heater, by performing feedback control of supplied electric power. In more detail, the supplying-of-power controller 116b controls operation for supplying electric power in such a manner that, while the electric power is supplied from the electric power source 111 to the heater 40, the duty ratio of the electric power pulse is adjusted according to pulse width modulation (PWM) control. Further, in the case that the temperature of the heater 40 is decreased, the supplying-of-power controller 116b performs operation for temporarily increasing the temperature of the heater 40 by increasing the duty ratio of the electric power pulse, and, further, making the temperature reach the target temperature defined in the heating profile. Since the above the operation comprises contents such as those explained above, the duty ratio can be represented as a wave form that is shown by the line 23 in the graph in Fig. 14 and relates to puff action performed by a user.

Regarding the duty ratio of the electric power that is the control value used in the supplying-of-power controller 116b, a predetermined threshold value has been set as a change condition and associated with the duty ratio in advance. Further, when a change condition such as that explained above is satisfied (especially, when the quantity of increase in the duty ratio has exceeded a threshold value), the puff judgment unit 116c can judge that a user's puff action has performed. In the example of the line 23a in the graph shown in Fig. 15, by identifying five parts in the line 23a, wherein each of the five identified parts is that where the duty ratio was increased relatively to that equal to or larger than a predetermined threshold value within a fixed period of time, it has been judged that five puff actions were performed by a user (puffs # 1-#5).

In this regard, the quantity of increase in the duty ratio may be calculated by obtaining difference between a smallest value (minimum value) and a largest value (maximum value) in a predetermined length of time. The threshold value may be set to a value that is experimentally defined. Further, the threshold value may be set during a manufacturing process, or at any timing after completion of the manufacturing process. For example, a condition that the duty ratio goes up 20% or more in one second may be set as the change condition, and a judgment indicating the matter that a puff action has been performed may be made when the above condition is satisfied.

The change condition may be set in such a manner that it is associated with the time elapsed since a start of operation for supplying electric power. For example, as shown in Fig. 10 and Fig. 12, in the case that time sections, such as the initial temperature-increasing section, the middle temperature-decreasing section, and the temperature re-increasing section, are related to the heating profile, different change conditions may be set and related to different time sections, respectively.

According to an experiment performed by the inventor of the present invention, it has been ascertained that there is a tendency, in the heating control based on the heating profile, that the variation width regarding decrease in temperature of the heater 40 relating to puff action performed by a user decreases as the control process proceeds further through the initial temperature-increasing section, the middle temperature-decreasing section, and the temperature re-increasing section in this order. Thus, it is preferable that the change condition be set in such a manner that the ratio of increase in the duty ratio for compensating for decrease in temperature of the heater 40 by feedback control becomes smaller as the time elapsed since a start of operation for supplying electric power becomes longer. For example, with respect to the change conditions, a condition that the duty ratio goes up "30%" or more may be associated with the initial temperature-increasing section, a condition that the duty ratio goes up "25%" or more may be associated with the middle temperature-decreasing section, and a condition that the duty ratio goes up "20%" or more may be associated with the temperature re-increasing section.

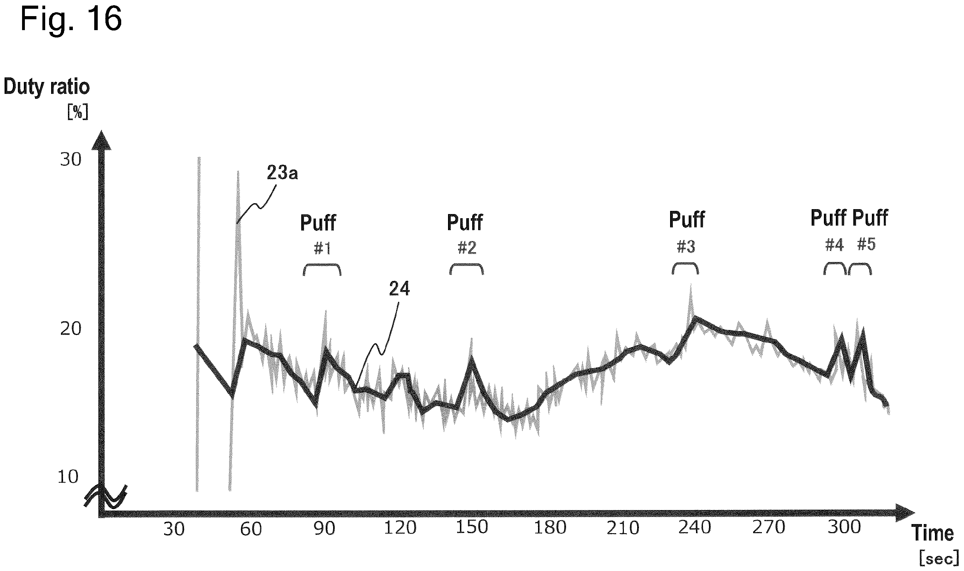

Fig. 16 is a graph which shows an example of chronological change in the moving average of the duty ratio in operation for supplying electric power. The line 24 in the graph shown in Fig. 16 shows an example of chronological change in the moving average that corresponds to the chronological data of the duty ratio represented by the line 23a in the graph in Fig. 15. In this regard, in Fig. 16, the line 24 in the graph is shown in such a manner that the line 24 is superimposed on the line 23a in the graph in Fig. 15.

The moving average has been generally known as a method for making chronological data smooth. In the moving average, average values of fixed sections are obtained, respectively, and chronological change in the average is shown. As a result, it becomes possible to further accurately determine the tendency of chronological change in the duty ratio. In this regard, a person skilled in the art will understand that any moving average method such as a simple moving average method, a weighted moving average method, an exponential moving average method, or the like may be used in the present embodiment.

As explained above, the ratio of increase in the moving average value with respect to the chronological data of the duty ratio may be applied in relation to the quantity of increase in the above-explained duty ratio. In an example, an average value of the duty ratio for each predetermined section (for example, 1 second) is obtained, and a judgment as to whether the average value of the duty ratio has went up 20% or more during a period including consecutive plural sections is made. In relation to the section that has been judged as that satisfying the above condition, the puff judgment unit 116c can judge that a user's puff action has been performed. That is, by judging occurrence of a user's puff action by using the rate of increase in the moving average value relating to the chronological data of the duty ratio, it becomes possible to avoid misjudgment and make the accuracy of judgment more stable.

### (2-7) Flow of Control Processing

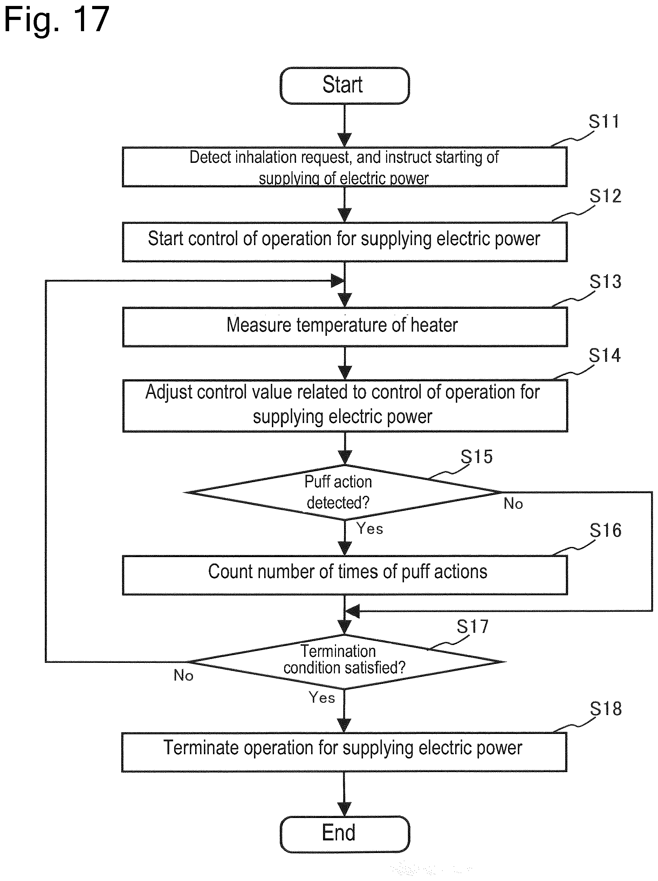

In the following description, an example of the flow of control processing performed by the inhaler 100 will be explained. Fig. 17 is a flow chart which shows an example of the flow of processing in a control method according to the present embodiment. Specifically, heating control relating to an aerosol source is performed based on the heating profile; that is, an example of the flow of processing, that is performed when control of operation for supplying electric power is being performed, for judging whether user's puff action has been performed will be shown. The processes in the present embodiment are performed by the controller 116.

It should be reminded that the respective processing steps explained in the following description are mere examples, so that the processing steps are not limited to those explained below; thus, a different step(s) may optionally be added or a processing step(s) may be omitted. Further, the order that the processing steps is to be performed, that is explained below, is also a mere example, so that there may be cases wherein the order is not limited to that explained below and the processing steps may be arranged in any order, or the processing steps are performed in a parallel manner.

As shown in Fig. 17, when the present process is started, the controller 116 first makes the sensor 112 detect a request for inhalation. In response thereto, the supplying-of-power instructing unit 116a instructs the electric power source 111 to start supplying of electric power to the heater 40 (step S11). The inhalation request corresponds to user manipulation performed by a user for requesting generating of aerosol. An example of the inhalation request is user manipulation applied to the inhaler 100, such as an action of a user for pressing a button installed in the inhaler 100.

The other example of the inhalation request is an action of a user for inserting the stick-type base material 150 into the inhaler 100. Detecting of a state that the stick-type base material 150 is being held by the holding part 140 may be performed by a pressure sensor installed in the holding part 140 by detecting contact pressure between the pressure sensor and the held stick-type base material 150. In more detail, when the stick-type base material 150 is inserted and held in the inside, the pressure sensor arranged on an inner surface of the holding part 140 and the outer periphery of the stick-type base material 150 are brought into contact with each other. In addition, the controller 116 makes the pressure sensor detect the contact pressure, so that a judgement indicating the state that stick-type base material 150 is being held by the holding part 140 can be made.

In a different example, the state that stick-type base material 150 is being held by the holding part 140 may be detected by using a capacitance-type proximity sensor arranged in a position near the opening 142, i.e., it may be detected based on change in capacitance or permittivity that is observed in relation to insertion of the stick-type base material 150 into the inside. In more detail, the capacitance-type proximity sensor arranged in a position near the opening 142 detects capacitance, permittivity, or the like in a subspace near the opening 142 in the inner space 141. In relation to inserting/extracting of the stick-type base material 150, various kinds of parts of the stick-type base material 150 pass through the subspace. In relation to the above movement, the quantities of capacitance and permittivity in the subspace changes. That is, the controller 116 can make a judgment indicating the state that the stick-type base material 150 is being held by the holding part 140, according to chronological change in capacitance or permittivity in a subspace such as that explained above.

Next, the supplying-of-power controller 116b performs operation for starting heating control that relates to the aerosol source and is based on the heating profile, that is, starts control of operation for supplying electric power (step S12). After step S12, the heating control performed by the supplying-of-power controller 116b is continued until control for supplying electric power is terminated in step S18.

The supplying-of-power controller 116b continuously measures the temperature of the heater 40 for heating control based on the heating profile (step S13). As explained above, the temperature of the heater 40 can be measured by using the quantity of change in the value of the quantity of electric power supplied to the heater 40 or the electric resistance value of the heat-generating resistor.

The temperature of the heater 40 measured in step S13 is controlled based on the heating profile that defines chronological change in target temperature of the heater 40. That is, the supplying-of-power controller 116b adjusts, based on the heating profile, the control value relating to operation for supplying electric power in such a manner that the temperature of the heater 40 is adjusted to reach the target temperature (step S14). As explained above, target temperatures may be set to correspond to time sections, respectively, wherein the respective time sections correspond to the respective lengths of time elapsed since a start of operation for supplying electric power. Further, it is preferable that the control value be adjusted in such a manner that the temperature of the heater 40 reaches a target temperature in an end stage in a time section corresponding to a length of elapsed time. Further, the control value may be a duty ratio of an electric power pulse, and the duty ratio of the electric power pulse may be adjusted by using pulse width modulation (PWM) control.

The puff judgment unit 116c judges that a puff action has been performed, if the control value used in step S14 satisfies a predetermined change condition (step S15). For example, the predetermined change condition that the control value may satisfy is a condition that the quantity of increase in the duty ratio is larger than a predetermined threshold value.

If it is judged in step S15 that a puff action has been performed (Yes), the puff counting unit 116d increments the number of times of puff actions performed by a user (step S16). On the other hand, if it is judged in step S15 that no puff action has been performed (No), step S16 is skipped.

The controller 116 repeats steps S13-S16 until a condition for terminating control is satisfied (step S17). For example, the termination condition may be a condition that the number of times of puff actions counted in step S16 in the period of the heating control reaches a predetermined number of times. It should be reminded that the termination condition is not limited to that explained above, and, in a different example, it may be a condition that the period of time during that the heating control was performed (i.e., the length of time of heating) exceeds a predetermined length of time.

Next, if it is judged in step S17 by the supplying-of-power controller 116b that the condition for terminating the heating control has been satisfied (Yes), the heating control based on the heating profile, i.e., control of operation for supplying electric power is terminated (step S18). The notification instructing unit 116e may make the notifier 113 output notification with respect to above termination.

Thereafter, i.e., after terminating of the heating control in step S18, the present processing is terminated.

As explained above, according to the present embodiment, puff action performed by a user can be incorporated, as a part in information processing, in the information processing realized by software. That is, it becomes possible to provide a method for judging whether a puff action has been performed, by using a simple method that does not require installation of a dedicated sensor in a device. As a result, disadvantages relating to manufacture, such as increasing of the size of the inhaler, increasing of the cost for manufacturing the inhaler, and so on, can be suppressed. Further, according to the present embodiment, the control value (for example, the duty ratio of the electric power pulse) used by the controller 116 in control of operation for supplying electric power from the electric power source 111 to the heater 40 can be applied as it stands as a parameter for judging a puff action. That is, puff action performed by a user can be judged in a simplified and accurate manner, for judging whether a puff action is performed by the user.

### « 3. Modification Examples »

### (3-1) First Modification Example

In the above-explained embodiment, heating control of an aerosol source, i.e., control of operation for supplying electric power, is performed based on a single heating profile. In addition to the above, or in place of the above, in a first modification example, plural kinds of heating profiles may be set in advance, and control processing based on each of the heating profiles may be performed. In the above case, the condition for judging whether puff action has been performed may be set flexibly in relation to each of the heating profiles.

In the following description, it is supposed, as an example, that plural kinds of heating profiles a and b are set in advance, and they are stored in the memory 114. Similar to the case shown in Fig. 10, each of the heating profiles a and b comprises an initial temperature-increasing section, a middle temperature-decreasing section, and a temperature re-increasing section. On the other hand, the chronological change in target temperature of the heater 40 defined in the heating profile a is different from that defined in the heating profile b. Examples of the above heating profiles a and b are shown in Table 3-1 and Table 3-2.

### (Table 3)

**Table 3-1. Example of Heating Profile a**

| Time Section | Length of Time | Target Temperature |
|---|---|---|
| Initial Temperature-Increasing Section | T₁ₐ seconds | TMP₁ₐ °C |
| Middle Temperature-Decreasing Section | (T₂ₐ-T₁ₐ) seconds | TMP₂ₐ °C |
| Temperature Re-increasing Section | (T_{end}-T₂ₐ) seconds | TMP₃ₐ °C |

**Table 3-2. Example of Heating Profile b**

| Time Section | Length of Time | Target Temperature |
|---|---|---|
| Initial Temperature-Increasing Section | T_{1b} seconds | TMP_{1b} °C (>TMP₁ₐ °C) |
| Middle Temperature-Decreasing Section | (T_{2b}-T_{1b}) seconds | TMP_{2b} °C (>TMP₂ₐ °C) |
| Temperature Re-increasing Section | (T_{end}-T_{2b}) seconds | TMP_{3b} °C (>TMP₃ₐ °C) |

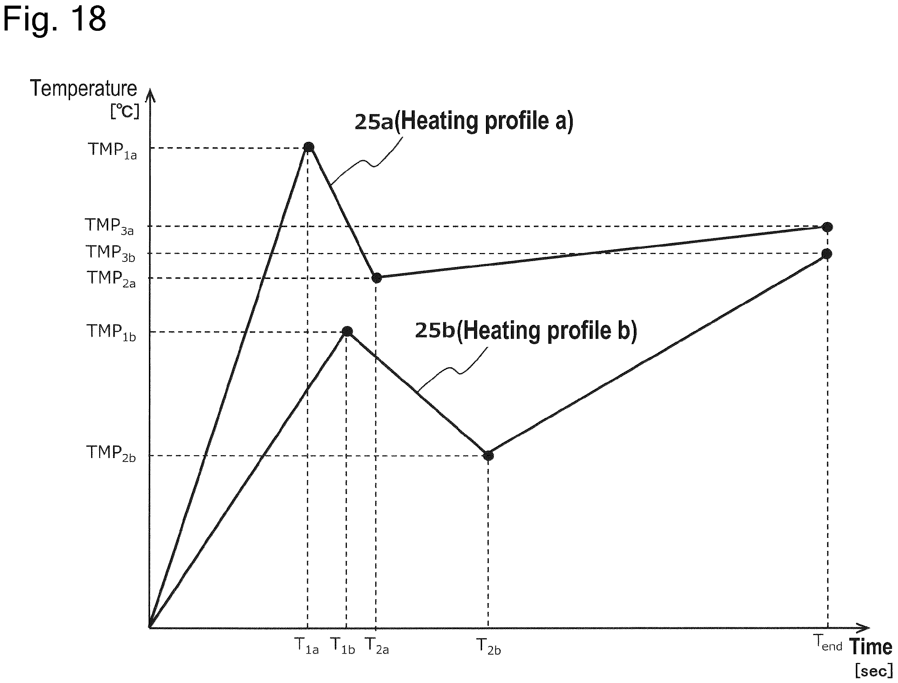

Fig. 18 is a graph which shows examples of chronological change in actual temperature of the heater 40 which was operated based on heat profiles a and b shown above. The horizontal axis of the graph represents time (seconds). The vertical axis of the graph represents temperature (degrees Celsius) of the heater 40. The lines 25a and 25b in the graph represent chronological change in actual temperature of the heater 40. As shown in Tables 3-1 and 3-2 and Fig. 18, the length of time of each section and the target temperature in each section relating to the heating profile a are different from those relating to the heating profile b. Especially, the heating profile a is set in such a manner that the temperature of the hater 40 becomes relatively higher through the whole part thereof, compared with that relating to the heating profile b.

In the present modification example, regarding the above-explained change condition that is used in judgment with respect to a puff action performed by a user, a change condition that is different from that related to the heating profile a may be related to the heating profile b. That is, a change condition A may be related to the chronological change in the heating profile a, and a change condition B, that is different from the change condition A, may be related to the chronological change in the heating profile b.

In this regard, the temperature of the heater 40 operated in relation to the chronological change in the higher-temperature heating profile changes in a temperature range higher than a temperature range of change in the temperature of the heater 40 operated in relation to the chronological change in the lower-temperature heating profile. The above matter means that the temperature difference between the temperature of the heater 40 and the temperature of the air taken in as a result of user's puff action becomes large. That is, the quantity of the temperature fall relating to a puff action becomes large when the chronological change in the higher-temperature heating profile is adopted, compared with that in the case that the chronological change in the lower-temperature heating profile is adopted.

Thus, in the case that the target temperature relating to the chronological change in the heating profile a is higher than that relating to the chronological change in the heating profile b as shown in Tables 3-1 and 3-2 and Fig. 18, it is preferable that the corresponding change condition A be set more strict than the change condition B. The matter that the change condition A is more strict than the change condition B includes, for example, the matter that the threshold value associated with the change condition A is set to that larger than that associated with the change condition B. For example, it is preferable to set the more restrict change condition A as a condition that the duty ratio goes up 20% or more, and, on the other hand, set the change condition B as a condition that the duty ratio goes up 15% or more.

That is, according to the present modification example, since it is possible to flexibly set, in relation to respective heating profiles, conditions used for judging whether puff action has been performed, it becomes possible to further improve and stabilize accuracy of judgment with respect to puff action.

### (3-2) Second Modification Example

In the above-explained embodiment, for judging whether user's puff action has been performed, a judgment method based on change in the control value (the duty ratio) used in operation for supplying electric power is adopted. In addition thereto, in a second modification example, in relation to judgment with respect to user's puff action, a different method based on change in temperature of the heater 40 is also adopted, i.e., two methods relating to judgment with respect to puff action are adopted together.

As explained above, it has been ascertained experimentally that there is a tendency, in the heating control based on the heating profile, that the variation width regarding decrease in temperature of the heater 40 relating to puff action performed by a user decreases as the control process proceeds further through the initial temperature-increasing section, the middle temperature-decreasing section, and the temperature re-increasing section in this order. That is, it can be assumed that accuracy of judgment may become unstable if a single method only is used for judgment with respect to puff action. Further, for example, in the case that the heating profile is defined as that including a section during that supplying of electric power is not performed, the duty ratio in the above section becomes zero, and, accordingly, a judgment method based on change in the duty ratio cannot be applied thereto.

Thus, in the present modification example, in addition to the puff action judging method according to the present embodiment that is based on change in the duty ratio, a different method is also applied, i.e., the both methods are used together, for further stabilizing accuracy of judgment with respect to puff action.

Specifically, in the present modification example, in addition to puff action judgment based on change in the duty ratio, the puff judgment unit 116c in the controller 116 performs, based on change in temperature of the heater 40, judgment with respect to user's puff action. The temperature of the heater 40 is measured by the sensor 112 which is a component of a temperature measuring part. That is, the puff judgment unit 116c is constructed to be able to make a judgment indicating a performed puff action in the case that the temperature of the heater 40 has satisfied a predetermined temperature change condition.

As explained above, the temperature of the heater 40 can be measured by arranging, for example, a temperature sensor (thermistor) in a position neat the heater 40, and change in the temperature of the heater 40 can be determined by using measured temperature. Further, instead of using a thermistor, it is possible to install a voltage sensor to detect an electric resistance value of a heat-generating resistor or the quantity of electric power supplied to a heating element, and, based on the quantity of change thereof, determine change in the temperature of the sensor 40. That is, it is possible to adopt a method for judging, based on determination as to whether the temperature of the heater 40 satisfies a predetermined temperature change condition, user's puff action.

In more detail, chronological change of target temperature of the heater 40 in the heating profile is related to at least two sections. Specifically, the above sections comprises a first-half section that corresponds to a period from a point in time when operation for supplying electric power starts to a point in time when a predetermined length of time has elapsed, and a latter-half section that corresponds to a period from a point in time when the first-half section ends to a point in time when a predetermined length of time has elapsed since the end of the first-half section. In the first-half section, the puff judgment unit 116c performs judgment with respect to puff action, based on the temperature of the heater 40, i.e., according to determination as to whether the temperature of the heater 40 satisfies the temperature change condition. On the other hand, in the latter-half section, the puff judgment unit 116c performs judgment with respect to puff action, according to determination as to whether the duty ratio satisfies the change condition.

That is, it is sufficient if operation for judging, based on the temperature of the heater 40, whether puff action has been performed is performed, in the first-half section during that the variation width regarding the quantity of temperature change relating to puff action is large. On the other hand, in the latter-half section during that the variation width regarding the quantity of temperature change becomes small, judgment accuracy in judging, based on the temperature of the heater 40, with respect to puff action can be further stabilized by performing judgment with respect to puff action based on the duty ratio.

In the case that the present modification example is applied to the example of control relating to the heating profile shown in Fig. 10, the first-half section may be defined as that comprising the initial temperature-increasing section and the middle temperature-decreasing section, and the latter-half section may be defined as that comprising the temperature re-increasing section. In this regard, in the heating control, the initial temperature-increasing section is a section for preheating the heater 40 after starting of operation for supplying electric power. The middle temperature-decreasing section is a section following the initial temperature-increasing section, and, during that the temperature of the heater 40 is reduced by stopping the operation for supplying electric power. The temperature re-increasing section is a section during that the heater is heated again by resuming the operation for supplying electric power.

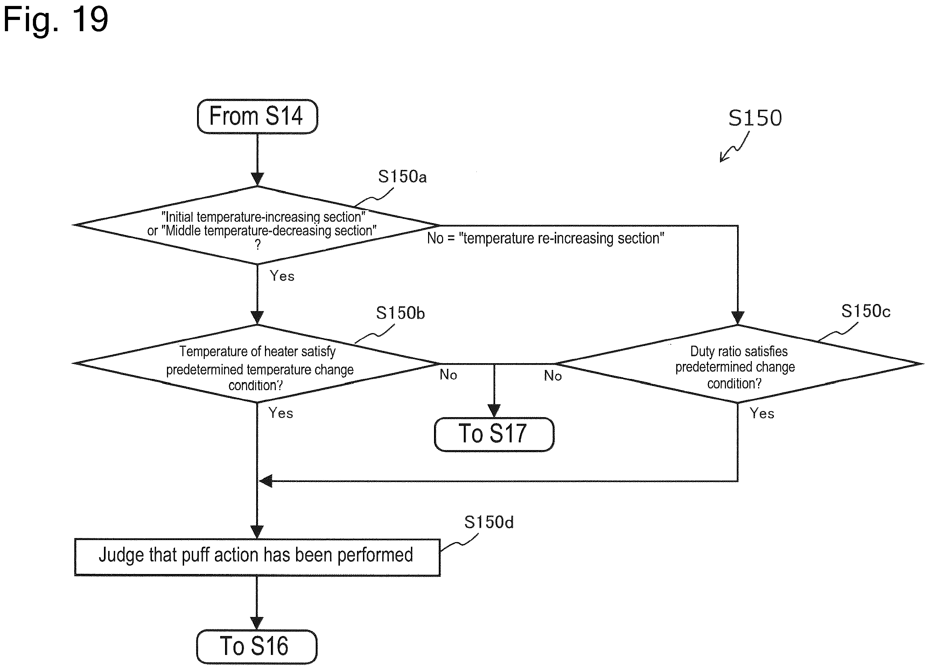

Fig. 19 is a flow chart which shows an example of the flow of control processing that is realized by applying the present modification example thereto. The present flow chart can be applicable as a modification example of step S15 in the flow chart in Fig. 17 that has been explained in relation to the above-explained embodiment.

In the present modification example, following step S17 in Fig. 17, judgment as to whether a section, that corresponds to the length of time elapsed until the present time since a start of control of operation for supplying electric power to the heater 40, is either one of the initial temperature-increasing section and the middle temperature-decreasing section (step S150a). If the time elapsed until the present time is that in the range of the initial temperature-increasing section or the middle temperature-decreasing section (Yes), operation for judging, based on the temperature of the heater 40, whether puff action has been performed is performed. That is, the puff judgment unit 116c may perform operation for judging whether the temperature of the heater 40 satisfies a temperature change condition (step S150b), and, if the temperature satisfies the temperature change condition (Yes), make a judgment indicating a performed puff action (step S150d).

On the other hand, if a section corresponding to the present elapsed time is the temperature re-increasing section (step S150a: No), operation for judging, based on the duty ratio, whether puff action has been performed is performed. That is, the puff judgment unit 116c may perform operation for judging whether the duty ratio satisfies a predetermined change condition (step S150c), and, if the duty ratio satisfies the predetermined change condition (Yes), make a judgment indicating a performed puff action (step S150d).

That is, according to the present modification example, plural methods for the judgment process with respect to puff action are prepared, and a method can be selected appropriately from the plural method when performing the judgment process with respect to puff action. As a result, accuracy of judgment with respect to puff action can be further improved and stabilized.

### (3-3) Third Modification Example

Similar to the second modification example, two methods relating to judgment with respect to puff action are used together in a third modification example; wherein a different method based on change in temperature of the heater 40 is adopted therein. Especially, in the present modification example, in the heating control relating to the heating profile, the method for judging, based on change in the duty ratio, whether puff action has been performed and the method for judging (detecting), based on change in the temperature of the heater 40, whether puff action has been performed are performed at the same time.

Specifically, throughout the heating control relating to the heating profile, the puff judgment unit 116c may make a judgment indicating performed puff action, in either one of or both the case that the duty ratio satisfies a predetermined change condition and the case that the temperature of the heater 40 satisfies a predetermined temperature change condition. In other words, the above two methods are performed concurrently, and a puff action performed by a user may actually be specified in the case that a judgment indicating a performed puff action is made based on one method and the same judgment is also made based on the other method. In a different construction, the above two methods are also performed concurrently, and puff action performed by a user may actually be specified in the case that the puff judgment unit 116c has made, based either one of the two method, a judgment indicating a performed puff action.

Thus, according to the present modification example, plural methods for the judgment process with respect to puff action are prepared, and the plural methods can be used concurrently when performing the judgment process with respect to puff action. As a result, accuracy of judgment with respect to puff action can be further improved.

### << 4. Supplement >>

In the above description, the preferred embodiments in the present disclosure, together with some modification examples, have been explained in detail with reference to the attached figures. According to the present embodiments, puff action performed by a user can be incorporated, as a part in information processing, in the information processing realized by software. That is, it becomes possible to provide a method for making a judgment with respect to puff action, by using a simple method that does not require installation of a dedicated sensor in a device. As a result, disadvantages relating to manufacture, such as increasing of the size of the inhaler 100, increasing of the cost for manufacturing the inhaler 100, and so on, can be suppressed. Further, according to the present embodiments, the control value (for example, the duty ratio of the electric power pulse) used by the controller 116 in control of operation for supplying electric power from the electric power source 111 to the heater 40 can be applied as it stands as a parameter for making a judgment with respect to puff action. That is, judgment as to whether a puff action has been performed by a user can be performed in a simplified and accurate manner, for judgment with respect to a puff action performed by the user.

It should be understood that the present disclosure is not limited to any of the above examples. It is obvious to a person, who has ordinary skill in the technical field to which the present invention pertains, that it is possible to conceive of various kinds of modified examples or rectified examples within the scope of the technical idea disclosed in the claims; and it is understood that they are also those pertaining to the scope of the technique of the present invention.

It should be reminded that the series of processes performed by the respective devices explained in the present specification may be realized by using any of software, hardware, or a combination of software and hardware. Programs, that are components of the software, are stored in advance in a storage medium/media (a non-transitory medium: a non-transitory media) implemented in the inside or the outside of the respective devices, for example. Further, for example, each program is read into a RAM when a computer, which controls each device explained in the present specification, is operated, and is executed by a processor such as a CPU. The storage medium is, for example, a magnetic disk, an optical disk, a magneto-optical disk, a flash memory, or the like. Further, the computer program may be delivered via a network without using a storage medium, for example.

It should be reminded that the constructions such as those shown below are also constructions within the scope of the techniques of the present invention.
(1) An inhaler comprising:
   a heater for heating a base material, which includes an aerosol source, for generating aerosol;
   an electric power source for supplying electric power to the heater;
   a temperature measuring unit for measuring temperature of the heater;
   a memory for storing information that defines chronological change in target temperature of the heater; and
   a controller for controlling operation for supplying the electric power, for making the temperature of the heater reach the target temperature;
   wherein the controller makes a judgment indicating that a puff action has been performed by a user, in the case that a control value related to the operation for supplying the electric power has satisfied a predetermined change condition.
(2) The inhaler explained in above item (1), wherein
   the target temperatures have been set to correspond to time sections, respectively, wherein each time section corresponds to the length of time elapsed since a start of the operation for supplying the electric power, and
   the controlling of the operation for supplying the electric power comprises adjusting of the temperature of the heater to make the temperature of the heater reach the target temperature in an end stage in the corresponding time section.
(3) The inhaler explained in above item (1) or (2), wherein the controlling of the operation for supplying the electric power comprises feedback controlling of supplied electric power for compensating for change in the temperature of the heater.
(4) The inhaler explained in any one of above items (1)-(3), wherein
   the controlling of the operation for supplying the electric power comprises adjusting of a duty ratio of an electric power pulse, according to pulse width modulation (PWM) control, and
   the control value is the duty ratio of the electric power pulse.
(5) The inhaler explained in above item (4), wherein the change condition is a condition that the quantity of increase in the duty ratio is greater than a predetermined threshold value.
(6) The inhaler explained in above item (5), wherein the quantity of increase in the duty ratio is the ratio of increase in a moving average value with respect to chronological data of the duty ratio.
(7) The inhaler explained in above item (5) or (6), wherein the predetermined threshold value is set in such a manner that it becomes smaller as the length of time elapsed since a start of the operation for supplying the electric power becomes longer.
(8) The inhaler explained in any one of above items (1)-(6), wherein the change condition is set in such a manner that it is related to the length of time elapsed since a start of the operation for supplying the electric power.
(9) The inhaler explained in any one of above items (1)-(8), wherein
   the chronological change in the target temperature comprises first-kind chronological change and second-kind chronological change,
   the change condition comprises a first change condition and a second change condition, and
   the first change condition is related to the first-kind chronological change, and the second change condition is related to the second-kind chronological change.
(10) The inhaler explained in above item (9), wherein the first change condition is set as that more strict than the second change condition, in the case that the target temperature in the first-kind chronological change is set as that higher than the target temperature in the second-kind chronological change.
(11) The inhaler explained in any one of above items (1)-(10), wherein the controller is further constructed to make a judgment indicating that a puff action has been performed, in the case that the temperature of the heater satisfies a predetermined temperature change condition.
(12) The inhaler explained in above item (11), wherein the temperature measuring unit is constructed to measure the temperature of the heater by using a temperature sensor arranged in a position near the heater.
(13) The inhaler explained in above item (11) or (12), wherein the controller is further constructed to make a judgment indicating that a puff action has been performed, in either one of or both the case that the control value has satisfied the predetermined change condition and the case that the temperature of the heater has satisfied the predetermined temperature change condition.
(14) The inhaler explained in any one of above items (11)-(13), wherein
   the chronological change is related to
      a first section that corresponds to a period from a point in time when the operation for supplying the electric power starts to a point in time when a first length of time has elapsed, and
      a second section that corresponds to a period until a point in time when a second length of time has elapsed after the end of the first section; and
   the controller performs
      judgment, that is based on the temperature of the heater, in the first section to indicate that a puff action has been performed, in the case that the temperature of the heater has satisfied the predetermined temperature change condition, and
      judgment, that is based on the control value, in the second section to indicate that a puff action has been performed, in the case that the control value has satisfied the predetermined change condition.
(15) The inhaler explained in above item (14), wherein, in the chronological change,
   the first section comprises
      an initial temperature-increasing section for preheating the heater after a start of the operation for supplying the electric power, and
      a middle temperature-decreasing section, that follows the initial temperature-increasing section, for lowering the temperature of the heater by stopping the operation for supplying the electric power; and
   the second section comprises
      a temperature re-increasing section for again heating the heater by resuming the operation for supplying the electric power.
(16) The base material used in the inhaler explained in any one of above items (1)-(15).
(17) A method for controlling an inhaler, wherein
   the inhaler comprises
      a heater for heating a base material, which includes an aerosol source, for generating aerosol, and
      an electric power source for supplying electric power to the heater; and
   the method includes:
      measuring temperature of the heater;
      adjusting, based on information that defines chronological change in target temperature of the heater, a control value related to operation for supplying the electric power, for making the temperature of the heater reach the target temperature; and
      making a judgment indicating that a puff action has been performed by a user, in the case that a control value has satisfied a predetermined change condition,
   wherein the above steps are those performed during the operation for supplying the electric power.
(18) The method explained in above item (17), wherein
   the target temperatures have been set to correspond to time sections, respectively, wherein each time section corresponds to the length of time elapsed since a start of the operation for supplying the electric power, and
   the control value is adjusted to make the temperature of the heater reach the target temperature in an end stage in the corresponding time section.
(19) The method explained in above item (17) or (18), wherein the adjusting the control value comprises feedback controlling of supplied electric power for compensating for change in the temperature of the heater.
(20) The method explained in any one of above items (17)-(19), wherein
   the control value is a duty ratio of an electric power pulse,
   the adjusting the control value comprises adjusting of the duty ratio according to pulse width modulation (PWM) control, and
   the change condition that is to be satisfied by the control value is a condition that the quantity of increase in the duty ratio is greater than a predetermined threshold value.
(21) The method explained in above item (20), wherein the quantity of increase in the duty ratio is the ratio of increase in a moving average value with respect to chronological data of the duty ratio.
(22) The method explained in any one of above items (17)-(22) further comprising
   incrementing the counted number of times of puff actions performed by a user, in relation to making a judgment indicating that a puff action has been performed by a user, and
   terminating the operation for supplying the electric power when the counted number of times of puff actions has reached a predetermined number of times.
(23) A program which makes a computer perform the method explained in any one of items (17)-(22).

### REFERENCE SIGNS LIST

100 Inhaler: 111 Electric power source: 112 Sensor: 113 Notifier: 114 Memory: 115 Communicator:
116 Controller: 116a Supplying-of-power instructing unit: 116b Supplying-of-power controller: 116c Puff judgment unit: 116d Puff counting unit: 116e Notification instructing unit:
40 heater: 150 Stick-type base material:

## Claims

1. An inhaler comprising:
a heater for heating a base material, which includes an aerosol source, for generating aerosol;
an electric power source for supplying electric power to the heater;
a temperature measuring unit for measuring temperature of the heater;
a memory for storing information that defines chronological change in target temperature of the heater; and
a controller for controlling operation for supplying the electric power, for making the temperature of the heater reach the target temperature;
wherein the controller makes a judgment indicating that a puff action has been performed by a user, in the case that a control value related to the operation for supplying the electric power has satisfied a predetermined change condition.

2. The inhaler according to Claim 1, wherein
the target temperatures have been set to correspond to time sections, respectively, wherein each time section corresponds to the length of time elapsed since a start of the operation for supplying the electric power, and
the controlling of the operation for supplying the electric power comprises adjusting of the temperature of the heater to make the temperature of the heater reach the target temperature in an end stage in the corresponding time section.

3. The inhaler according to Claim 1 or 2, wherein the controlling of the operation for supplying the electric power comprises feedback controlling of supplied electric power for compensating for change in the temperature of the heater.

4. The inhaler according to any one of Claims 1-3, wherein
the controlling of the operation for supplying the electric power comprises adjusting of a duty ratio of an electric power pulse, according to pulse width modulation (PWM) control, and
the control value is the duty ratio of the electric power pulse.

5. The inhaler according to Claim 4, wherein the change condition is a condition that the quantity of increase in the duty ratio is greater than a predetermined threshold value.

6. The inhaler according to Claim 5, wherein the quantity of increase in the duty ratio is the ratio of increase in a moving average value with respect to chronological data of the duty ratio.

7. The inhaler according to Claim 5 or 6, wherein the predetermined threshold value is set in such a manner that it becomes smaller as the length of time elapsed since a start of the operation for supplying the electric power becomes longer.

8. The inhaler according to any one of Claims 1-6, wherein
the change condition is set in such a manner that it is related to the length of time elapsed since a start of the operation for supplying the electric power.

9. The inhaler according to any one of Claims 1-8, wherein
the chronological change in the target temperature comprises first-kind chronological change and second-kind chronological change,
the change condition comprises a first change condition and a second change condition, and
the first change condition is related to the first-kind chronological change, and the second change condition is related to the second-kind chronological change.

10. The inhaler according to Claim 9, wherein the first change condition is set as that more strict than the second change condition, in the case that the target temperature in the first-kind chronological change is set as that higher than the target temperature in the second-kind chronological change.

11. The inhaler according to any one of Claims 1-10, wherein the controller is further constructed to make a judgment indicating that a puff action has been performed, in the case that the temperature of the heater satisfies a predetermined temperature change condition.

12. The inhaler according to Claim 11, wherein the temperature measuring unit is constructed to measure the temperature of the heater by using a temperature sensor arranged in a position near the heater.

13. The inhaler according to Claim 11 or 12, wherein the controller is further constructed to make a judgment indicating that a puff action has been performed, in either one of or both the case that the control value has satisfied the predetermined change condition and the case that the temperature of the heater has satisfied the predetermined temperature change condition.

14. The inhaler according to any one of Claims 11-13, wherein
the chronological change is related to
a first section that corresponds to a period from a point in time when the operation for supplying the electric power starts to a point in time when a first length of time has elapsed, and
a second section that corresponds to a period until a point in time when a second length of time has elapsed after the end of the first section; and
the controller performs
judgment, that is based on the temperature of the heater, in the first section to indicate that a puff action has been performed, in the case that the temperature of the heater has satisfied the predetermined temperature change condition, and
judgment, that is based on the control value, in the second section to indicate that a puff action has been performed, in the case that the control value has satisfied the predetermined change condition.

15. The inhaler according to Claim 14, wherein, in the chronological change,
the first section comprises
an initial temperature-increasing section for preheating the heater after a start of the operation for supplying the electric power, and
a middle temperature-decreasing section, that follows the initial temperature-increasing section, for lowering the temperature of the heater by stopping the operation for supplying the electric power; and
the second section comprises
a temperature re-increasing section for again heating the heater by resuming the operation for supplying the electric power.

16. The base material used in the inhaler recited in any one of Claims 1-15.

17. A method for controlling an inhaler, wherein
the inhaler comprises
a heater for heating a base material, which includes an aerosol source, for generating aerosol, and
an electric power source for supplying electric power to the heater; and
the method includes:
measuring temperature of the heater;
adjusting, based on information that defines chronological change in target temperature of the heater, a control value related to operation for supplying the electric power, for making the temperature of the heater reach the target temperature; and
making a judgment indicating that a puff action has been performed by a user, in the case that a control value has satisfied a predetermined change condition,
wherein the above steps are those performed during the operation for supplying the electric power.

18. The method according to Claim 17, wherein
the target temperatures have been set to correspond to time sections, respectively, wherein each time section corresponds to the length of time elapsed since a start of the operation for supplying the electric power, and
the control value is adjusted to make the temperature of the heater reach the target temperature in an end stage in the corresponding time section.

19. The method according to Claim 17 or 18, wherein
the control value is a duty ratio of an electric power pulse,
the adjusting the control value comprises adjusting of the duty ratio according to pulse width modulation (PWM) control, and
the change condition that is to be satisfied by the control value is a condition that the quantity of increase in the duty ratio is greater than a predetermined threshold value.

20. The method according to any one of Claims 17-19 further comprising
incrementing the counted number of times of puff actions performed by a user, in relation to making a judgment indicating that a puff action has been performed by a user, and
terminating the operation for supplying the electric power when the counted number of times of puff actions has reached a predetermined number of times.
